Europäisches Patentamt

European Patent Office

Office européen des brevets

(19)

(11) Numéro de publication: **0 261 009 B1**

# (12) FASCICULE DE BREVET EUROPEEN

(45) Date de publication de fascicule du brevet: **22.06.94**

(21) Numéro de dépôt: **87401901.1**

(22) Date de dépôt: **18.08.87**

(51) Int. Cl.⁵: **C12N 15/00**, C12N 1/20, C12N 9/00, C12P 21/02, C07K 7/10, //(C12N1/20, C12R1:125),(C12P21/02, C12R1:125)

(54) **Sequence d'ADN exercant une fonction se manifestant par une surproduction de protéines exocellulaires par diverses souches de bacillus, et vecteurs contenant cette séquence.**

(30) Priorité: **18.08.86 FR 8611826**

(43) Date de publication de la demande:
**23.03.88 Bulletin 88/12**

(45) Mention de la délivrance du brevet:
**22.06.94 Bulletin 94/25**

(84) Etats contractants désignés:
**AT BE CH DE ES FR GB GR IT LI LU NL SE**

(56) Documents cités:
EP-A- 0 117 823
EP-A- 0 227 260

CHEMICAL ABSTRACTS, vol. 105, 1986, page 142, résumé no. 1447z, Columbus, Ohio,US; M. YANG et al.: "Identification of the pleiotropic sacO gene of Bacillus subtilis"

CHEMICAL ABSTRACTS, vol. 104, no. 19, mai 1986, page 164, résumé no. 163023q, Columbus, Ohio, US

(73) Titulaire: **INSTITUT PASTEUR**
**25/28, rue du Docteur Roux**
**F-75015 Paris(FR)**

(72) Inventeur: **Amory, Antoine Philippe**
**5 Square Le Nain**
**F-78960 Voisins-le-Bretonneux(FR)**
Inventeur: **Rapoport, Georges**
**20 rue de l'Armorique**
**F-75015 Paris(FR)**
Inventeur: **Kunst, Frederik**
**46 rue Barbès, Bâtiment 18**
**F-94200 Ivry-sur-Seine(FR)**
Inventeur: **Klier, André**
**6 allée Guynemer**
**F-93330 Neuilly-sur-Marne(FR)**
Inventeur: **Dedonder, Raymond**
**3 allée des Pépinières**
**F-92290 Chatenay-Malabry(FR)**

EP 0 261 009 B1

CHEMICAL ABSTRACTS, vol. 102, 1985, page 136, résumé no. 90798t, Columbus, Ohio, US; J. OKADA et al.: "Cloning of the gene responsible for the extracellular proteolytic activities of Bacillus licheniformis"

BIOTECHNOLOGY, vol. 3, no. 11, novembre 1985, page 967, Londres, GB; A.S.WEISS: "Researchers cultivate new uses for bacilli"

GENE, vol. 46, no. 2/3, 1986, pages 247-255, Amsterdam, NL; M.M. ZUKOWSKI et al.: "Hyperproduction of an intracellular heterologous protein in a sacUh mutant of Bacillus subtilis"

CHEMICAL ABSTRACTS, vol. 106, no. 25, 22 juin 1987, page 181, résumé no. 208793a, Columbus, Ohio, US; M. DEBARBOUILLE et al.: "Cloning of the sacS gene encoding a positive regulator of the sucrose regulon in Bacillus subtilis"

CHEMICAL ABSTRACTS, vol. 106, no. 15, 13 avril 1987, page 143, résumé no.114364j, Columbus, Ohio, US; A. AMORY et al.: "Characterization of the sacO genes from Bacillus licheniformis and Bacillus subtilis"

(74) Mandataire: **Gutmann, Ernest et al**
**Ernest Gutmann - Yves Plasseraud S.A.**
**3, rue Chauveau-Lagarde**
**F-75008 Paris (FR)**

## Description

L'invention concerne, d'une façon générale, une séquence d'ADN exerçant une fonction se manifestant par une surproduction de protéines exocellulaires par diverses souches de Bacillus, ainsi que des vecteurs contenant cette séquence.

L'invention concerne plus particulièrement une séquence d'ADN codant pour un polypeptide agissant sur la surproduction d'enzymes exocellulaires, notamment de protéase alcaline, par B.subtilis.

En effet, l'intérêt croissant porté ces derniers temps à l'utilisation de B.subtilis en tant qu'hôte pour le clonage, destiné notamment à la production de protéines exocellulaires, s'explique par les motifs suivants. Tout d'abord, la caractérisation génétique de B.subtilis a été largement effectuée, et des méthodes efficaces d'introduction et d'extraction de fragments d'ADN ainsi que des marqueurs sélectifs de résistance à certains antibiotiques ont déjà été décrits. D'autre part, grâce à ses propriétés de sporulation, B.subtilis représente un bon modèle de différenciation procaryotique.

D'un point de vue pratique, une des propriétés intéressantes de cet organisme est que l'on peut le manipuler en parfaite sécurité puisqu'il n'infecte ni les humains , ni les animaux.

D'autre part, il possède la propriété de secréter un grand nombre d'enzymes (Bacteriological reviews ; 41 , 711-753,(1977), dont certaines, telles que l'α-amylase, les protéases ou les glucanases, sont particulièrement intéressantes au niveau industriel . En particulier, la protéase alcaline est très utilisée dans la fabrication de détergents ainsi que dans l'industrie pharmaceutique et dans l'industrie agro-alimentaire.

Les techniques de recombinaison de l'ADN ont également permis l'utilisation de B. subtilis comme hôte permettant la sécrétion de protéines étrangères. Dans ces constructions, le gène correspondant à la protéine en question est fusionné avec une séquence permettant son expression ainsi que sa sécrétion par B.subtilis, ladite séquence étant encore désignée par l'expression "séquence signal" codant pour un peptide signal. De nombreux exemples ont été décrits qui utilisent les séquences régulatrices ainsi que les régions correspondant aux peptides signal de l'α-amylase, de la protéase alcaline ou de la protéase neutre de B. subtilis (J. Bacteriol., 165, 837-842, (1986)), B. amyloliquefaciens ou B. licheniformis.

Toutefois, bien que B. subtilis semble être un hôte récepteur de choix par sa caractérisation bien établie, les niveaux de sécrétion de cet organisme sont beaucoup plus faibles si on les compare à ceux des souches de Bacillus utilisées dans l'industrie telles que B.licheniformis, B.amyloliquefaciens ou B.stearothermophilus qui sont capables de produire de grandes quantités d'enzymes sécrétées.

Un exemple caractéristique est celui de la protéase alcaline.

La souche de B.subtilis 168 mise en culture dans des conditions de laboratoire et dans un milieu de culture optimum peut produire 50 à 100 unités d'azocaséine par ml de surnageant d'activité du type de celle de la protéase alcaline.

La souche industrielle B.licheniformis (déposée à la Collection Nationale des Cultures Micro-organismes à l'Institut Pasteur de Paris sous le numéro 71 I 001) cultivée dans le même milieu de culture optimum que précédemment peut produire plus de 100 000 unités d'azocaséine par ml, donc avec un rendement multiplié par un facteur de 1000.

Il se peut que de telles souches aient accumulé des mutations altérant les propriétés de l'enzyme sécrétée telles que la thermostabilité, et/ou des mutations augmentant les taux de sécrétion et de synthèse de protéines.

Au cours de ces dernières années, des recherches approfondies ont été réalisées afin d'augmenter la production de protéase alcaline chez la souche bien caractérisée de B.subtilis 168. C'est ainsi qu'un grand nombre de mutants permettant l'augmentation des niveaux de sécrétion d'enzymes protéolytiques ont été isolés.

Premièrement, des mutants hpr ont été caractérisés par leur surproduction de protéases alcalines et neutres (J. Bacteriol. ; 112 : 1026-1028, (1972)).

Des mutants sac U[h] (Biochimie ; 56 : 1481-1489, (1974)), identiques aux mutants pap (J.Bacteriol. ; 161 : 1182-1187 (1985), et J.Bacteriol. ; 124 : 48-54, (1975)), ont également été décrits comme possédant un phénotype pléïotropique de surproduction de lévane-saccharase, de protéase et d'α-amylase.

En outre, le locus régulateur nprR2 a été décrit comme étant responsable de la stimulation spécifique de la sécrétion de la protéase neutre (J.Bacteriol. ; 139 : 583-590,(1979), et J.Bacteriol. ; 119 : 82-91, (1974)). Toutefois, les bases moléculaires de ces mutations restent toujours inconues puisqu'aucune des formes alléliques mutées de ces gènes impliqués dans le phénotype observé n'a pu être clonée. C'est la raison pour laquelle le transfert de ces mutations aux autres espèces de Bacillus de façon à augmenter leur capacité de production est très difficile.

Plusieurs équipes de recherche ont récemment décrit le clonage de fragments d'ADN permettant l'hypersécrétion de protéase chez B.subtilis.

Le brevet japonais déposé le 29/07/83 par Okada et al sous le n° 60-30 685 décrit un ADN recombinant contenant une séquence d'insertion originaire du génome d'un B.licheniformis présenté comme codant pour une protéase de ce dernier type de micro-organisme. Les auteurs du brevet ont utilisé cet ADN recombinant pour transformer des cultures de Bacillus subtilis en vue d'accroître leur capacité à produire des protéases alcalines et neutres.

Okada et al (Appl.Microbiol.Biotechnol. ; 20 : 406-412, (1984)) ont cloné un fragment EcoRI de 3,6 kb à partir de B.licheniformis qui stimule aussi bien la sécrétion de la protéase neutre que celle de la protéase alcaline (70x), mais n'a qu'un faible effet sur les enzymes du type α-amylase (2x) ; le phénotype observé est conservé à l'occasion du clonage d'un fragment PvuII de 1,25 kb et même d'un fragment Sau 3A1 de 0,37 kb obtenu à partir du fragment EcoRI. Les auteurs n'excluent pas la possibilité que la séquence clonée appartenait à un gène régulateur, plutôt qu'à des gènes codant des protéases.

Tomioka et al (J.Biotechnol., 3 : 85-96, (1985)) ont aussi décrit la stimulation de la sécrétion des protéases neutres (10x) et alcalines (9x) avec un fragment EcoRI de 4 kb provenant de B.amyloliquefaciens ; ce fragment a pu être réduit à 1,75 kb comprenant des phases ouvertes de lecture non identifiées ; il ne permet pas la stimulation de sécrétion d'α-amylase.

Un fragment d'ADN issu de Bacillus natto a été décrit comme permettant une hyperproduction de protéases alacalines et neutres et de lévane-saccharase (J. Bacteriol., 166, 20-29, (1986)). Les auteurs ont envisagé qu'un polypeptide de 60 acides aminés pouvait intervenir dans la stimulation de cette hyperproduction.

Enfin, l'identification du gène sacQ, gène pleïotropique affectant l'expression d'un grand nombre de gènes codant pour des produits secrétés chez diverses souches de Bacillus ainsi que l'identification de polypeptides de 46 acides aminés codés par ce gène sac Q ont récemment été décrites chez B. subtilis (J. Bacteriol., 166, 113-119, (1986)), et chez B. amyloliquefaciens (J. Biotechnol., 3, 85-96, (1985)).

C'est dans le but d'induire chez B. subtilis une surproduction supérieure à ce qui a été réalisé jusqu'à ce jour, non seulement de protéases alcalines, mais également d'un grand nombre d'enzymes exocellulaires, que les auteurs de ce brevet ont utilisé une approche différente des précédentes.

En effet, les inventeurs ont construit une banque de gènes à partir du chromosome de la souche surproductrice B.licheniformis, qu'ils ont transférés à B.subtilis, après ligature dans des plasmides appropriés, et ont sélectionné les colonies de B.subtilis surproductrices de protéases. Ils ont ainsi isolé un plasmide - ci-après dénomé pPR4 - contenant un fragment d'insertion ou "insérat" Sau3A1 de 3,5 kb qui confère à la cellule hôte la propriété de surproduire des enzymes secrétées, notamment de la protéase alcaline.

La réduction de cet insérat par digestion partielle par Sau3A1 a conduit les inventeurs à isoler un fragment de 1,4 kb qui, lorsqu'inséré dans un plasmide et introduit par l'intermédiaire de ce dernier dans une cellule hôte y induit la même propriété de surproduction que précédemment. Un plasmide caractéristique contenant ce fragment de 1,4 kb et qui a été utilisé dans l'étude approfondie de ce fragment, a été dénommé pPR41. La présence de ce fragment dans une souche de B.subtilis permet à cette dernière de produire 70 fois plus de protéase alcaline qu'elle ne le faisait auparavant, 4 fois plus de protéase neutre, et au moins 50 fois plus de lévane-saccharase. Elle confère également à la cellule hôte la propriété de surproduire d'autres enzymes telles que la cellulase, ou la β-glucanase, la xylanase, et l'α-amylase.

Au cours de cette description, il sera fait référence au dessin et aux reproductions photographiques dans lesquels :

- la figure 1 est représentative de la surproduction de protéase alcaline conférée par le plasmide pPR4 à une souche de B.subtilis,
- la figure 2 représente le vecteur pPR41 portant la séquence de 1,4 kb de l'invention,
- la figure 3 montre la séquence complète et la carte de restriction du fragment de 1,4 kb,
- les figures 4a à 4f fournissent des résultats comparatifs des surproductions de différentes enzymes par diverses souches porteuses d'un plasmide comprenant une séquence de l'invention ou non,
- la figure 5 montre les étapes de sous-clonage du fragment de 1,4 kb,
- la figure 6 représente les plasmides pQL1 et pQS1 contenant respectivement les gènes $sacQ_L$ et $sacQ_S$, sous contrôle du même promoteur inductible spac, et qui sont définis en page 8 de ce texte.
- la figure 7 indique les différences entre les polypeptides codés respectivement à partir du gène sacQ de B. subtilis, de B. amyloliquefaciens, et de B. licheniformis,
- la figure 8 représente les structures de promoteurs possibles dans les différentes souches de Bacillus.
- la figure 9 représente la construction du géne de fusion sacR-aph3'.
- la figure 10 représente la carte de restriction d'un fragment HindIII-EcoRI de B. subtilis contenant la séquence signal de la lévane-saccharase.

-   la figure 11 représente le plasmide pBS610 contenant le fragment d'ADN de la figure 10.
-   la figure 12 représente le plasmide pBS620 contenant également le fragment d'ADN de la figure 10.

Le phénotype d'hypersecrétion conféré à la souche réceptrice de B. subtilis par le plasmide pPR41 sera désigné dans ce qui suit par le symbole Ses$^h$ (synthèse d'enzymes secrétées, haut niveau).

L'étude de la séquence entière de ce fragment d'ADN de 1,4 kb montre qu'il n'existe aucune phase ouverte de lecture supérieure à 101 acides aminés. Les inventeurs ont donc exclu que ce fragment d'ADN de 1,4 kb contienne le gène de structure d'une protéase du type subtilisine, qui est composé de 379 acides aminés sous sa forme précurseur, et de 274 résidus sous sa forme mature (Nucl. Ac. Res., 13, 8913-8926, (1985)), et cherché à réduire la taille de ce fragment.

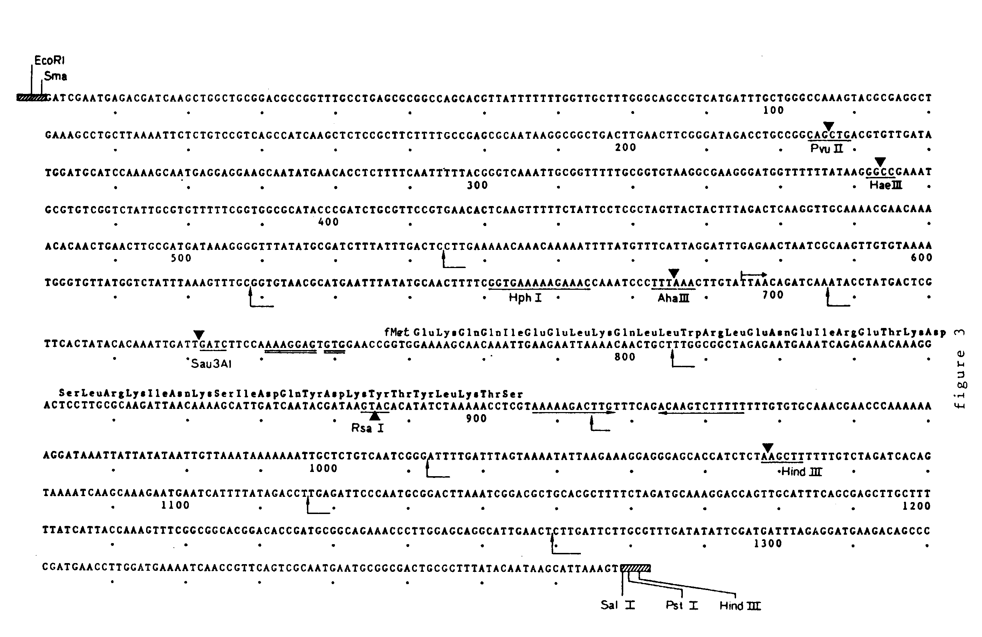

Le phénotype Ses$^h$ est conservé lorsque l'on substitue au fragment 1,4 kb, des fragments plus petits qui en sont issus, dans les plasmides utilisés pour la transformation de B. subtilis. De tels fragments plus petits peuvent être obtenus au terme de différentes délétions réalisées sur les extrémités 5' et 3' du fragment de 1,4 kb. C'est ainsi que les inventeurs ont constaté que la séquence responsable du phénotype Ses$^h$ est comprise dans un fragment de 561 paires de base entre les positions 353 et 914 de la fig. 3. Dans ce fragment, les 109 derniers nucléotides, correspondant aux positions 805 à 914, sont essentiels mais non suffisants pour produire l'hypersecrétion chez B. subtilis. Par contre, les 228 derniers nucléotides, correspondant aux positions 686 à 914, sont suffisants pour induire le phénotype Ses$^h$ à condition de faire précéder ce fragment par un promoteur de transcription reconnu par B. subtilis. Ce fragment de 228 paires de bases contient une phase ouverte de lecture qui code pour un polypeptide de 46 acides aminés, et qui est délimitée par les nucléotides situés aux positions 768 et 905. Cette phase ouverte de lecture est précédée par une séquence nucléotidique présumée fournir les signaux typiques de liaison au ribosome délimité par les nucléotides situés aux positions 751 et 762, et suivie d'un terminateur de transcription délimité par les nucléotides situés aux positions 907 et 936. Les inventeurs ont également déterminé que le promoteur de transcription se trouve sur le fragment de 333 paires de bases délimité par les nucléotides situés aux positions 353 et 686 de la figure 3.

Le polypeptide de 46 résidus codé par ce fragment de 228 paires de base de B. licheniformis (encore appelé polypeptide sacQ$_L$) présente une certaine homologie avec le produit du gène sacQ de B. subtilis (ou encore polypeptide sacQ$_S$) et de B.amyloliquefaciens (ou encore polypeptide sacQ$_A$) qui produisent le même genre de phénotype Ses$^h$ chez B. subtilis.

Toutefois, les inventeurs montrent que le polypeptide sacQ$_L$ exprimé par la séquence nucléotidique correspondante incorporée dans B. subtilis induit le phénotype (Ses$^h$) d'une façon bien plus efficace que ne le fait le polypeptide sacQ$_S$, ou sacQ$_A$ dans les mêmes conditions. Un dosage précis montre que l'activation de l'expression du gène de la protéase alcaline est trois fois meilleure, alors que l'activation du gène sacB (lévane-saccharase) est 5 fois meilleure. Cette meilleure efficacité du polypeptide de B. licheniformis est probablement liée à sa structure primaire.

Des expériences de délétion ont été réalisées par les inventeurs à partir du polypeptide sacQ$_L$ de 46 acides aminés. Ils ont ainsi déterminé que la portion carboxyterminale dudit polypeptide, et plus particulièrement les 7 derniers acides aminés, peuvent être modifiés, voire supprimés, sans entraîner pour autant une perte totale de l'expression du phénotype Ses$^h$, et que le peptide constitué des seuls 12 premiers acides aminés est inactif.

Il semble donc qu'une partie au moins des résidus compris entre le 12ème et le 39ème résidu du polypeptide sacQ$_L$ et correspondant au fragment d'ADN délimité par les nucléotides situés aux positions 805 et 885 de la figure 3, soit essentielle pour l'expression du phénotype Ses$^h$.

L'expression accrue du phénotype Ses$^h$ lors de l'introduction dans une souche de B. subtilis d'un vecteur comprenant une séquence d'ADN codant pour le polypeptide sacQ$_L$ pourrait être le résultat de l'action sur une séquence cible, comprise dans le génome de B. subtilis, soit du polypeptide sacQ$_L$ lui-même, soit d'un autre produit exprimé par une partie du génome de B. subtilis sous le contrôle de sacQ$_L$.

Les séquences cibles sus-mentionnées sont généralement situées en amont des gènes de structure codant pour les enzymes secrétées par B. subtilis.

Plus particulièrement, les inventeurs ont caractérisé une séquence cible comprise dans un fragment de 440 paires de bases, délimité par les sites de restriction Sau3A1-Rsa1 et situé en amont du site de liaison aux ribosomes et de la séquence d'ADN codant pour la lévane-saccharase. Cette séquence cible est donc située en amont du gène sacB et comprend le gène sacR de B. subtilis.

L'invention concerne un fragment d'ADN caractérisé par le fait qu'il code pour le polypeptide ayant la structure suivante :

```
(Nterminal)Met-Glu-Lys-Gln-Gln-Ile-Glu-Glu-Leu-Lys-Gln-
Leu-Leu-Trp-Arg-Leu-Glu-Asn-Glu-Ile-Arg-Glu-Thr-Lys-Asp-
Ser-Leu-Arg-Lys-Ile-Asn-Lys-Ser-Ile-Asp-Gln-Tyr-Asp-Lys-
Tyr-Thr-Tyr-Leu-Lys-Thr-Ser(Cterminal),
```

ou pour tout fragment de ce peptide qui conserve la propriété du peptide susdit, d'activer l'expression du phénotype Ses[h], notamment de stimuler la production des enzymes sus-mentionnées des souches de B. subtilis.

Les conditions dans lesquelles des fragments actifs peuvent être identifiés ont déjà été illustrées plus haut.

De préférence, le fragment d'ADN selon l'invention, est caractérisé par la structure nucléotidique s'étendant entre les positions 768 et 905 de la figure 3.

L'invention concerne également des séquences d'ADN dans lesquelles les susdits fragments sont précédés des séquences de régulation de l'expression de la séquence codant pour le susdit polypeptide et suivis des séquences nucléotidiques comprenant les éléments terminateurs de transcription. Ces éléments peuvent consister dans les séquences de régulation et/ou de terminaison qui sont associés avec le fragment d'ADN sus-défini dans les gènes naturels de B. licheniformis. A ce titre, l'invention concerne toute séquence d'ADN de plus grande dimension issue du fragment Sau3A1 de 3,5 kb mentionné plus haut et ne contenant pas de séquence nucléotidique codant pour tout ou partie d'une protéase alcaline, cette séquence contenant également la séquence codant pour le polypeptide capable d'induire le phénotype Ses[h], ainsi que les séquences nucléotidiques comprenant les éléments de régulation de l'expression et/ou les éléments de terminaison de la transcription de ce dernier. En particulier, l'invention concerne des séquences d'ADN comprenant le fragment de 561 paires de bases délimité par les positions 353 et 914 de la séquence de 1,4 kb de la figure 3, susceptible d'être obtenue à partir du génome de B.licheniformis.

L'invention concerne plus particulièrement tout fragment du génome d'un Bacillus licheniformis contenu dans la séquence d'environ 1 400 nucléotides de l'acide nucléique de la figure 3 et comprenant la séquence codant pour le polypeptide activateur du phénotype Ses[h].

Le fragment d'acide nucléique du genre en question peut-être constitué par la totalité de la séquence de 1 400 nucléotides de l'acide nucléique de la figure 3. Cependant il a été observé que la totalité de la séquence n'est pas nécessaire.

A titre d'exemples de fragments conformes à l'invention et satisfaisant à l'ensemble des conditions qui ont été rappelées dans la définition sus-indiquée du fragment d'ADN selon l'invention, on mentionnera :

- le polynucléotide délimité par des nucléotides d'extrémités correspondant aux nucléotides situés aux positions 353 et 914 respectivement de la figure 3, qui comprend le promoteur de transcription, le site de liaison ribosomique, le gène de structure du polypeptide sacQ$_L$ de 46 acides aminés.
- Le polynucléotide délimité par des nucléotides d'extrémités correspondant aux nucléotides situés aux positions 353 et 1 012 respectivement de la figure 3, qui comprend en plus du polynucléotide précédent le terminateur de transcription,
- le polynucléotide contenant le fragment délimité par des nucléotides d'extrémités correspondant aux positions 686 à 914 respectivement de la figure 3, et comprenant, en outre, en amont dudit fragment, une séquence suffisamment longue pour également inclure le promoteur endogène permettant la transcription dans la cellule hôte, notamment par B. subtilis,
- tout polynucléotide comprenant un fragment compris entre le nucléotide 353, d'une part, et le nucléotide 905, et comprenant, en outre, en aval dudit fragment, une séquence suffisamment longue pour inclure les séquences de terminaison homologues du gène de B. licheniformis.
- tout polynucléotide délimité d'une part par un nucléotide compris entre les positions 1 à 353 et, d'autre part, par un nucléotide compris entre les positions 905 à 914 de la figure 3.
- tout polynucléotide délimité d'une part par un nucléotide compris entre les positions 1 à 353 et, d'autre part, par un nucléotide compris entre les positions 1012 à 1400 de la figure 3.

L'invention concerne aussi tout acide nucléique recombinant contenant tout fragment du type sus-indiqué codant pour le polypeptide SacQ$_L$ activateur du phénotype Ses[h] associé avec des fragments d'acide nucléique hétérologue vis-à-vis dudit fragment, c'est-à-dire des fragments d'acide nucléique ayant des séquences étrangères à celle des séquences d'acide nucléique qui, dans le génome de B.licheniformis, entourent le fragment défini ci-dessus.

A ce titre, l'invention concerne tout ADN recombinant contenant une séquence codant pour le polypeptide sacQ$_L$ activateur du phénotype Ses[h] respectivement associée avec un promoteur de transcription reconnu par B. subtilis, et/ou un terminateur de transcription hétérologue par rapport à l'ADN de B. licheniformis, ce promoteur et/ou ce terminateur étant néanmoins reconnu par les polymérases de B. subtilis.

A cet égard, l'invention concerne plus particulièrement les ADN recombinants consistant en des vecteurs, contenant un fragment d'ADN selon l'invention en l'un de leurs sites non essentiels pour leur réplication, notamment du type plasmide, qui sont aptes à se répliquer dans B.subtilis et à y permettre l'expression de la séquence codant pour le polypeptide sacQ$_L$ activateur du phénotype Ses$^h$.

Avantageusement, le susdit promoteur de transcription hétérologue est choisi parmi ceux dont l'activité peut être induite. De tels promoteurs sont appelés dans ce qui suit promoteurs inductibles. A titre d'exemple de promoteur inductible, on citera le promoteur spac (Proc. Natl. Acad. Sci. USA, 81, 439-443, (1984)) inductible par l'IPTG (isopropyl $\beta$-D-thiogalactoside).

Des vecteurs préférés selon l'invention sont des vecteurs bifonctionnels capables de se répliquer aussi bien chez E.coli que chez B.subtilis, notamment les plasmides navette pHV33 ou pMK4 décrits respectivement dans Plasmid ; 6 : 193-201 (1981) et Gene ; 29 : 21-26, (1984), la ligature des séquences de l'invention aux génomes de ces vecteurs se faisant alors au niveau des sites Bam HI de ces derniers. pMK4 est illustratif des plasmides multicopies qui font également partie de l'invention, c'est-à-dire des plasmides capables de se multiplier en un grand nombre de copies dans la cellule hôte.

Les vecteurs selon l'invention sont donc préférentiellement choisis parmi ceux permettant d'induire un surproduction du polypeptide sacQ$_L$ dans la cellule hôte, tels que les plasmides multicopies sus-mentionnés comprenant un promoteur inductible.

L'invention concerne également le polypeptide sacQ$_L$ de 46 acides aminés sus-défini et les fragments peptidiques actifs qui en découlent.

Ce polypeptide selon l'invention est défini comme l'activateur de l'expression d'un, ou de plusieurs gènes cibles dans un organisme donné.

La structure primaire de l'activateur peut être modifiée dans sa partie carboxyterminale sans perte trop importante de son activité, notamment les sept derniers acides aminés peuvent être remplacés par d'autres résidus.

L'invention concerne un procédé de production d'enzymes secrétées, qui comprend la transformation de B. subtilis au moyen des plasmides sus-indiqués, la mise en culture des cellules de B. subtilis transformées dans un milieu approprié et la récupération desdites enzymes à partir du milieu de culture

Un tel procédé selon l'invention permet la surproduction d'un grand nombre d'enzymes exocellulaires, dont essentiellement la protéase alcaline, mais aussi la levansaccharase, la carboxymethylcellulase, la $\beta$-glucanase, la lichénase, la xylanase, l'$\alpha$-amylase par des souches de B.subtilis.

L'invention vise également un procédé de production d'un polypeptide déterminé par une souche de B. subtilis dont le génome a été modifié par insertion d'une séquence nucléotidique codant pour ledit polypeptide, qui comprend la transformation de la souche de B. subtilis sus-indiquée, au moyen de vecteurs permettant la surproduction d'un activateur du phénotype Ses$^h$ dansles cellules de B. subtilis , la mise en culture desdites cellules transformées dans un milieu approprié, et la récupération dudit polypeptide à partir du milieu de culture.

Parmi les activateurs du phénotype Ses$^h$ sus-indiqués, on citera notamment les polypeptides sacQ$_L$, sacQ$_S$, sacQ$_A$, et le polypeptide de 60 acides aminés issu de B. natto sus-mentionnés.

Préférentiellement, les vecteurs utilisés pour la mise en oeuvre d'un tel procédé sont les plasmides sus-indiqués permettant la surproduction de sacQ$_L$ chez B. subtilis.

D'une manière générale, toute enzyme naturellement secrétée par B. subtilis est tout d'abord traduite sous forme d'un polypeptide précurseur dont la séquence en acides aminés correspond à celle d'un peptide signal suivie de celle de l'enzyme secrétée. Au moment de la secrétion cellulaire, seule la forme mature correspondant à l'enzyme elle-même est secrétée hors de la cellule.

La modification du génome de B. subtilis, préalablement à la transformation de ce dernier selon le procédé de l'invention, est préférentiellement réalisée de manière à ce que le susdit génome comprenne une séquence nucléotidique codant pour un polypeptide déterminé, précédée par une séquence nucléotidique, ci-après désignée par "séquence signal", codant pour un peptide signal, et dont le rôle est de permettre la secrétion dudit polypeptide, ladite séquence signal étant elle-même précédée par une séquence nucléotidique comprenant une séquence cible précédemment définie et un promoteur de l'expression d'un tel fragment d'ADN.

Ainsi le remplacement d'une séquence d'ADN codant pour la production intracellulaire d'une enzyme chez B. subtilis par une séquence d'ADN codant pour un polypeptide déterminé, ou le remplacement d'une séquence d'ADN codant pour la sécrétion d'une enzyme chez B. subtilis, par une séquence d'ADN codant pour un peptide signal lié au polypeptide déterminé, permet d'obtenir ledit polypeptide directement secrété sous sa forme définitive par la cellule hôte, après transformation de cette dernière par un plasmide contenant une séquence d'ADN codant pour un polypeptide activateur du phénotype Ses$^h$ selon le procédé de l'invention.

La souche de B. subtilis utilisée pour la mise en oeuvre d'un tel procédé est avantageusement modifiée au moyen d'un vecteur, notamment plasmide, contenant, en l'un de ses sites non essentiels pour sa réplication, la séquence d'ADN codant pour le polypeptide déterminé, précédée d'une séquence signal, qui est elle-même précédée par une séquence nucléotidique comprenant une séquence cible et les éléments nécessaires pour promouvoir l'expression de la séquence signal et de la séquence codant pour le polypeptide déterminé.

L'invention a également pour objet un procédé de production d'un polypeptide déterminé par une souche de B. subtilis qui comprend la transformation de ladite souche de B. subtilis au moyen d'un vecteur contenant, en l'un de ses sites non essentiels pour sa réplication, une séquence d'ADN codant pour un polypeptide activateur du phénotype Ses$^h$, et permettant la surproduction dudit activateur dans les cellules de B. subtilis, et une séquence d'ADN codant pour le polypeptide déterminé, cette dernière séquence d'ADN étant précédée par une séquence signal qui est elle-même précédée par une séquence nucléotidique comprenant une séquence cible et les éléments nécessaires pour promouvoir l'expression de la séquence signal et de la séquence codant pour le polypeptide déterminé, la mise en culture desdites cellules transformées dans un milieu approprié, et la récupération dudit polypeptide à partir du milieu de culture.

Parmi les activateurs du phénotype Ses$^h$ sus-indiqués, on citera notamment les polypeptides sacQ$_L$, sacQ$_S$, sacQ$_A$, et le polypeptide de 60 acides aminés issu de B. natto sus-mentionnés.

Avantageusement, le vecteur sus-mentionné comprend une séquence d'ADN codant pour le polypeptide activateur sacQ$_L$.

Parmi les séquences signal utilisées, pour la construction des vecteurs de l'invention, on citera notamment celle permettant la secrétion de la protéase alcaline, de la lévane-saccharase, de la cellulase et de l'$\alpha$-amylase.

D'une manière générale, toute séquence signal précédant la séquence d'ADN codant pour une enzyme par B. subtilis peut être utilisée.

Préférentiellement dans le cas où le polypeptide déterminé risque d'être dégradé, sous l'action d'une ou plusieurs des enzymes naturellement secrétées par B. subtilis, notamment de la protéase alcaline, le gène codant pour une telle enzyme est modifié par délétion ou par mutation, de manière à ce que ladite enzyme ne soit pas secrétée, ou secrétée, sous forme inactive vis-à-vis du polypeptide déterminé.

Avantageusement, la séquence codant pour le polypeptide déterminé, dans les vecteurs sus-mentionnés, est précédée par la séquence signal comprise dans le gène sacB codant pour la lévane-saccharase chez B. subtilis , et qui est décrite dans Biochem. Biophys. Res. Com., 119, 2, 795-800 (1984), ladite séquence signal étant elle-même précédée par un fragment d'ADN contenant d'une part la séquence nucléotidique endogène Sau3A1-Rsa1 de 440 paires de bases sus-définie comprenant le gène sacR de B. subtilis et dans laquelle se situe la séquence cible et, d'autre part, les éléments nécessaires pour l'expression des séquences codant pour le peptide signal et pour le polypeptide déterminé.

En effet, la lévane-saccharase est secrétée par B. subtilis lorsque ce dernier est en phase exponentielle de croissance alors que les autres enzymes sont secrétées par B. subtilis en phase stationnaire. Ainsi l'insertion d'une séquence d'ADN codant pour un polypeptide déterminé en aval de la séquence signal endogène de la lévane-saccharase permet d'obtenir ledit polypeptide exempt des autres enzymes secrétées par B. subtilis lorsque la culture de ce dernier est arrêtée en fin de phase exponentielle de croissance.

L'invention concerne également un procédé de production d'un polypeptide déterminé pratiquement pur, qui comprend la mise en culture de cellules de B. subtilis modifiées à l'aide d'un plasmide contenant, en l'un de ses sites non essentiels pour sa réplication, une séquence d'ADN codant pour l'activateur sacQ$_L$, permettant la surproduction dudit activateur dans les cellules de B. subtilis, et une séquence d'ADN codant pour le polypeptide déterminé, cette dernière séquence d'ADN étant précédée par la séquence signal comprise dans le gène sacB codant pour la lévane-saccharase chez B. subtilis, ladite séquence signal étant elle-même précédée par un fragment d'ADN contenant d'une part la séquence nucléotidique endogène Sau3A1-Rsa1 de 440 paires de base comprenant le gène sacR de B. subtilis et dans laquelle se situe la séquence cible, et d'autre part les éléments nécessaires pour l'expression des séquences codant pour le peptide signal et pour le polypeptide déterminé, l'arrêt de la culture en fin de phase exponentielle de croissance desdites cellules de B. subtilis et la récupération du polypeptide déterminé à partir du milieu de culture pratiquement exempt des autres enzymes exocellulaires susceptibles d'être secrétées par les cellules de B. subtilis.

8

Il est avantageux d'utiliser des souches de B.subtilis porteuses de la mutation sacU^h, connue comme conférant à ces dernières une capacité de production accrue d'enzymes. L'introduction d'une séquence de l'invention chez des mutants sacU^h de B.subtilis, entraîne un effet de synergie affectant la surproduction des enzymes secrétées, voire d'un polypeptide déterminé dans le cas où les génomes des susdits mutants ont été modifiés par insertion d'une séquence codant pour ledit polypeptide corne il l'a été précédemment décrit.

A titre d'exemple de polypeptide déterminé susceptible d'être obtenu par le procédé selon l'invention, on citera les interférons humains, les interleukines et l'hormone de croissance.

Bien entendu, l'homme du métier appéciera que les polypeptides recherchés, plus particulièrement la protéase alcaline, ou le polypeptide déterminé, peuvent être purifiés de toute façon en soi connue, par exemple par électrophorèse sur gel et récupération du polypeptide recherché. On peut également encore avoir recours à toute autre technique par exemple à la chromatographie liquide sous haute pression.

Dans ce qui précède, n'a essentiellement été évoqué que la transformation de B.subtilis. L'invention n'est pas limitée à la transformation de ces cellules. Elle peut également être utilisée pour stimuler la production de polypeptides chez d'autres souches déjà utilisées dans l'industrie. On mentionnera, par exemple, B.licheniformis, B.amyloliquefaciens, B.stearothermophilus ou encore des souches E.coli.

Des caractéristiques supplémentaires de l'invention apparaitront encore au cours de la description qui suit des vecteurs préférés et des conditions dans lesquelles ils peuvent être utilisés, étant entendu que cette description ne saurait être interprétée comme tendant à restreindre la portée des revendications.

Etude du clonage des différents fragments d'ADN de l'invention ainsi que de leurs propriétés de surproduction enzymatique.

1) Matériel et méthodes

- Souches bacteriennes et plasmides :

Les souches bacteriennes utilisées sont Bacillus licheniformis (déposé à la C.N.C.M à l'Institut Pasteur de Paris sous le numéro 71 I 001) qui possède une forte activité de production de protéase extracellulaire, B. subtilis 512 qui est un mutant de la souche Marburg (J. Appl. Bacteriol., 33, 220-227,(1970)) déficient en protéase neutre, B. subtilis 512ts19 possédant une mutation spoOA thermosensible supplémentaire (Biochimie, 58, 109-117, (1976)), B.subtilis 1A510 (leuA8, arg15, thr5, recE4, stp) (J. Bacteriol., 156, 934-936, (1983)), B. subtilis QB881 (sacA321, leu8, argA), B. subtilis QB136 (sacU32, leu8, trpC2) (Biochimie, 56, 1481-1489, (1983)), E.coli MC1061 (araD139, Δ(ara,leu), 7697, ΔlacX74, galU⁻, galK⁻, hsr⁻, hsm⁺, strA) (J. Mol. Biol., 138, 179-207,(1980)) et JM83 (aro, Δlac, pro⁻, strA, thi, φ80, dlacZ, ΔM15) (Gene, 19, 259-268, (1982)).

Les plasmides vecteurs sont le pHV33 et le pMK4 précedemment cités, tous les deux étant capables de se répliquer chez E.coli et chez B. subtilis.

Les modifications de structure d'E.coli et de B. subtilis, nécesaires au transfert des génomes de plasmides sus-indiqués à ceux de ces bactéries, ont été réalisées, pour E.coli, par la procédure conventionnelle au chlorure de calcium, et pour B. subtilis par la procédure d'ANAGNOSTOPOULOS et SPIZIZEN (J. Bacteriol., 81, 741-746, (1961)).

- Milieux et tests qualitatifs

E.coli, tout comme B. subtilis, ont été mis en culture dans un bouillon L (10 g de Difco Bacto Tryptone, 5 g d'extrait de levure Difco, 5 g de chlorure de sodium par litre) ; le milieu SP est composé de 8 g/l de bouillon nutritif Difco Bacto, 1 mM de MgSO₄ et 13 mM de KCl auxquels on ajoute après stérilisation 2,5 µM de FeSO₄, 500 µM CaCl₂ et 10 µM de MnCl₂.

Le chloramphénicol est additionné à 5 µg/ml et l'ampicilline à 100 µg/ml. Le milieu minéral (MM) est composé de 60 mM de K₂HPO₄, 44 mM de KH₂PO₄, 3 mM de citrate de sodium, 2 mM de MgSO₄, 10 µM de MnCl₂, 0,5 mM de CaCl₂ et 10 mg/L de citrate d'ammonium ferrique (J. Bacteriol., 81, 741-746).

L'activité saccharolytique est estimée par détection de l'apparition de glucose avec du Statzyme Glucose 50 (Worthington).

Les hydrolyses de la carboxyméthylcellulose (Type II, Sigma), du lichénane (Sigma) et du xylane (Sigma) ont été détectées en recouvrant les boîtes de culture avec du rouge Congo 1 % pendant 1 heure et en lavant plusieurs fois avec du NaCl 1M jusqu'à ce que des zones claires apparaissent.

Les boîtes contenant des colonies qui ne se lient pas fermement à l'agar ont été recouvertes de 5 ml d'une solution d'agar mou à 7 g/l avant d'être colorées.

La dégradation de l'amidon est détectée en sublimant de l'iode sur les boîtes.

- Mesures des activités de la protéase neutre et de la protése alcaline :

Des dilutions appropriées de surnageants de culture ont été préincubées en présence ou en l'absence de 6 mM de fluorure de phénylméthanesulfonyle (PMSF). Des échantillons de 200 $\mu$l ont été ensuite additionnés à 1 ml d'une solution de Tris/HCl 100 mM (pH 8,0), 2 mM de CaCl$_2$, et 5 mg/ml d'azocaséine (Calbiochem) et incubés à 40°C. Les réactions sont arrêtées à l'aide de 0,8 ml de TCA 15 % (acide trichloroacétique). Après centrifugation, 1 ml de surnageant est neutralisé avec 50 $\mu$l de NaOH 10N et la densité optique est mesurée à 440 nm.

Une unité azocaséine correspond à 1 $\mu$g d'azocaséine hydrolysée par minute, ou encore 0,406 unité Delft.

L'activité protéasique sensible au PMSF est attribuée à la protéase alcaline, tandis que l'activité résistante au PMSF est due essentiellement à la protéase neutre.

- Mesure de l'activité saccharolytique

Une aliquote des surnageants de culture est incubée en présence de 8 % de saccharose, KH$_2$PO$_4$ 50 mM (pH 6,0) dans un volume final de 0,6 ml à 37°C. Les réac-tions sont arrêtées par ébullition. La quantité de glucose libérée est mesurée par incubation en présence d'orthodianisidine, glucose oxydase (dépourvue d'activité invertase) et péroxydase. Une unité enzymatique correspond à 1 $\mu$mole de glucose libérée par minute (Methods in Enzymol., 8, 500-505, (1966)).

2) Résultats

- Isolement d'un plasmide responsable de la surproduction de protéase.

L'ADN chromosomique de Bacillus licheniformis souche 71 I 001 a été partiellement coupé par Sau3A1 et des fragments de 3 à 5 kb ont été purifiés par électrophorèse sur gel d'agarose.

Ces fragments de chromosone ont été ligaturés a ceux du vecteur bifonctionnel pHV33 (E.coli - B.subtilis) au niveau du site déphosphorylé BamHI et le mélange de ligature a été utilisé pour transformer une souche d'E.coli MC 1061. Seize mille transformants contenant 85 % de recombinants ont été sélectionnés sur ampicilline et chloramphénicol.

L'ADN plasmidique a été extrait à partir de 8 groupes de 2 000 colonies chacun, purifié et utilisé pour transformer des cellules compétentes de B.subtilis 512.

La souche 512 est déficiente en activité du type protéase neutre et ne produit pas de halo caractéristique de précipitation de la caséine dû à l'activité de la protéase sécrétée.

Six mille colonies résistantes au chloramphénicol ont été testées pour leur activité du type protéase sur boîte de caséine à partir de chacun des 8 groupes.

Deux clones positifs ont été trouvés dans un des groupes.

L'ADN plasmidique a été extrait des 2 clones et utilisé pour transformer B.subtilis 512.

Tous les transformants obtenus présentent le même halo caractéristique autour des colonies, montrant ainsi que la surproduction de protéase est liée à la présence du plasmide dans B.subtilis.

Des analyses des 2 plasmides ont montré qu'ils étaient identiques et avaient inséré un fragment Sau3A1 du chromosome de 3,5 kb.

Un des plasmides a été appelé pPR4 et a été étudié plus en détail.

- Caractérisation de l'activité du type protéase

Le plasmide pPR4 ainsi que le vecteur PHV33 ont été introduits par transformation dans la souche 1A510 de B.subtilis qui est déficiente en fonctions de recombinaison mais qui porte les allèles sauvages des gènes protéases.

Les deux types de transformants obtenus ont été mis en culture sur des milieux SP contenant 1 % de caséine et 5 $\mu$g/ml de chloramphénicol.

L'activité protéase du surnageant de culture a été mesurée en présence ou en absence de PMSF qui inhibe spécifiquement la sérylprotéase (protéase alcaline).

L'activité PMSF résistante est due à la protéase neutre.

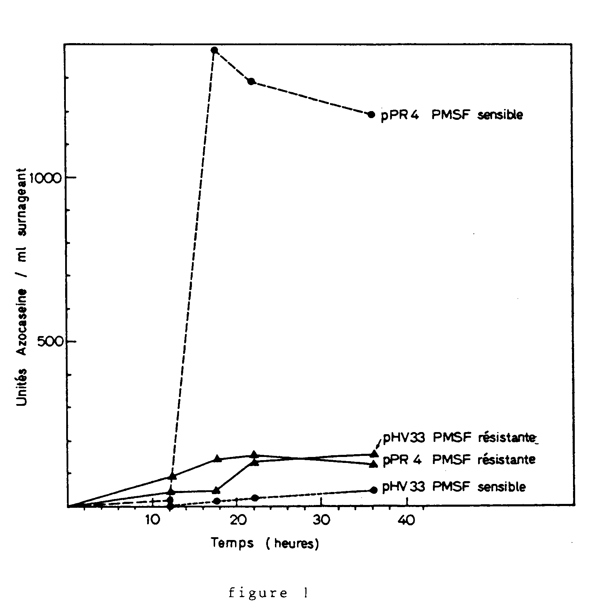

Comme l'indique la figure 1, une très forte stimulation de l'activité protéase alcaline est obtenue en présence du plasmide pPR4, en comparaison à la souche de référence portant le vecteur seul pHV33. Le maximum de stimulation qui est de 70 fois est obtenu après 17 heures de culture.

Toutefois, l'activité du type protéase neutre est beaucoup moins stimulée. L'augmentation maximale obtenue est d'un facteur de 4 après 17 heures de culture.

Après 22 heures de culture, il n'y a pas de différence d'activité protéase neutre entre les cellules contenant pPR4 ou pHV33.

- Sous-clonage du fragment de 3 500 paires de base

Le plasmide pPR4 qui porte le fragment de 3,5 kb a été partiellement digéré avec Sau3A1.

Les fragments obtenus ont été soumis à une électrophorèse sur gel d'agarose et 6 zones de migration correspondant aux tailles de fragment suivantes ont été découpées du gel :

1,2 à 1,4 kb ; 1,4 à 1,6 kb ; 1,6 à 1,8 kb ; 1,8 à 2,0 kb ; 2,0 à 2,3 kb et 2,3 à 2,8 kb.

L'ADN de chaque tranche du gel a été purifié par électroélution et ligaturé au niveau du site déphosphorylé BamHI du plasmide pMK$_4$.

Le vecteur pMK4 est un vecteur navette pour B.subtilis et E.coli obtenu par fusion de pC194 et pUC9.

La souche E.coli JM83 a été transformée avec le mélange de ligature et l'ADN plasmidique a été extrait à partir de tous les transformants s'étant multipliés en présence d'ampicilline et de chloramphénicol.

L'ADN extrait a été utilisé pour transformer B.subtilis 512ts19 (mutant de sporulation thermosensible de la souche 512), et les transformants ont été sélectionnés sur des boîtes de milieu minimum (MM) auquel est ajouté 2 % de lait écrémé et de chloramphénicol.

Des colonies surproductrices de protéases ont été trouvées pour chacun des 6 fragments de tailles différentes sélectionnés.

Une colonie de B.subtilis surproductrice de protéase correspondant à l'ADN issu de la tranche de gel comprise entre 1,2 à 1,4 kb ligaturée à pMK4 a été réisolée et son plasmide a été extrait et amplifié chez E.coli JM83 (pPR41).

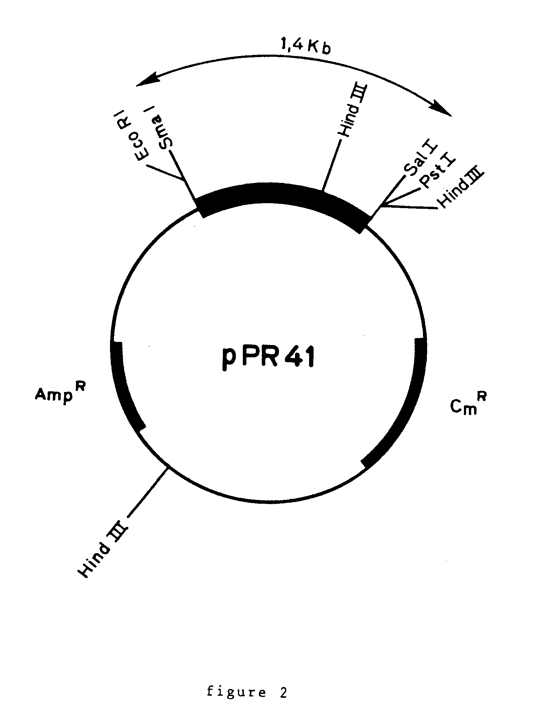

Le plasmide pPR41 contient un fragment d'insertion de 1,4 kb comme l'indique la figure 2.

La séquence de ce fragment de 1,4 kb a été dé-terminée et est indiquée à la figure 3.

Cette séquence a été traduite dans les 6 phases de lecture possibles. Aucun des polypeptides obtenus par traduction de cette séquence ne dépasse 101 acides aminés. Aucune homologie n'a été trouvée entre ces polypeptides et les protéases alcalines et neutres connues chez B. subtilis ou B. amyloliquefaciens. Nous pouvons donc exclure que le fragment de 1 400 paires de base contienne le gène de structure d'une protéase du type subtilisine, qui est composé de 379 acides aminés sous sa forme précurseur, et de 274 résidus sous sa forme mature (Nucl. Ac. Res., 13, 8913-8926, (1985)).

- Caractérisation du phénotype d'hypersécrétion (Ses[h]) :

Différents types d'activités enzymatiques secrétées testées sont augmentées en présence du plasmide pPR41. Le plasmide pPR41 ou le vecteur pMK4 ont été introduits par transformation dans différentes souches de B. subtilis présentées au tableau 1.

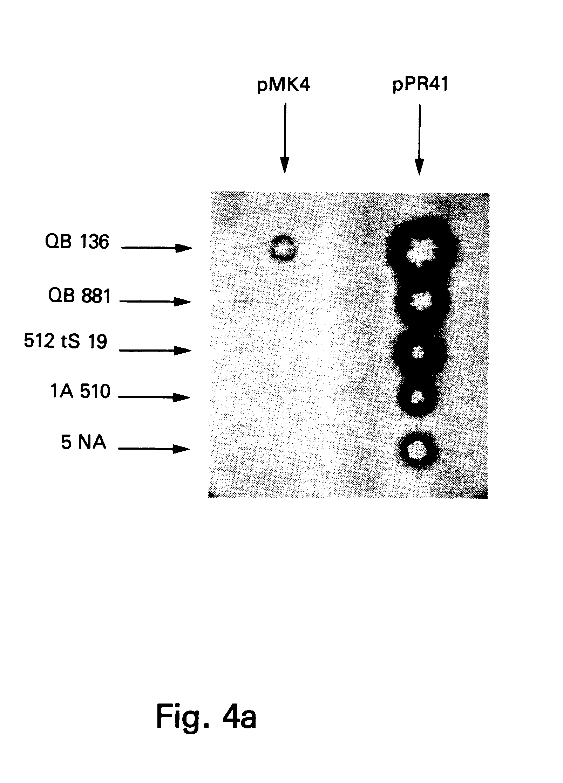

Les souches portant le plasmide recombinant ont été comparées à celles portant le vecteur seul, ces souches ayant été mises en culture sur des boîtes contenant différents substrats dégradables décrits dans les figures 4a à 4f.

Tableau 1

| SOUCHE | DESCRIPTION |
|--------|-------------|
| QB136 | Mutant SacU$^h$. Mutation pléiotropique qui augmente simultanément les niveaux de lévane− saccharase et de protéase extracellulaire. |
| QB881 | Mutant SacA⁻. Absence d'activité saccharolytique endocellulaire. Incapacité d'utiliser le saccharose comme source de carbone. |
| 512ts19 | Absence d'activité protéase neutre exocellulaire. Mutation spoOA thermosensible. |
| 1A510 | Rec⁻, Stp (stable transformation phenotype). Une plus grande stabilité des plasmides est décrite pour cette souche. |
| 5NA | Mutation spo0A. Incapacité de sporuler. |

L'activité protéase a été testée sur des boîtes contenant du lait écrémé en tant que source de carbone et d'azote. Toutes les souches testées présentent des niveaux d'activité de protéase extracellulaire augmentés (figure 4a). Cette stimulation est indépendante de l'intégration chromosomique du fragment cloné, puisqu'elle est également obtenue avec des souches Rec⁻ (B.subtilis 1A510), ou Rec⁺.

On trouve même une augmentation de la sécrétion de protéase chez des mutants spoOA, (B.subtilis 5NA), bien que cet effet ne soit pas aussi important qu'avec les autres souches. Les mutants spoOA sont bloqués au stade 0 (zéro) de la sporulation. Ils présentent normalement de très faibles niveaux de sécrétion et sont incapables d'exprimer le jeu entier des gènes de sporulation. Toutefois, la présence de pPR41 ne restaure pas le processus de sporulation de B.subtilis et l'on peut par conséquent exclure la fonction de complémentation de la mutation spoOA par pPR41.

La souche 512ts19, porteuse d'une mutation au niveau du gène de structure de la protéase neutre, présente également une sécrétion accrue d'enzyme protéolytique, confirmant ainsi les résultats de la figure 1 qui montrent que c est essentiellement la sécrétion de la protéase alcaline qui est stimulée.

Une découverte plus interessante encore est illustrée sur la figure 4a : le plasmide pPR41 semble stimuler la sécrétion de protéase chez un mutant sacU$^h$, (B.subtilis QB136). La mutation sacU$^h$ est connue comme étant une mutation pléiotropique qui augmente la secrétion d'un grand nombre d'enzymes exocellulaires, dont les protéases. Le plasmide pPR41 produit ainsi un effet synergique avec la mutation sacU$^h$ sur la secrétion de protéases chez B.subtilis.

Le taux de dégradation exocellulaire de cinq autres substrats différents est augmenté par la présence du plasmide pPR41 : hydrolyse du saccharose, dégradation de la carboxyméthylcellulose, du lichenane, du xylane et de l'amidon.

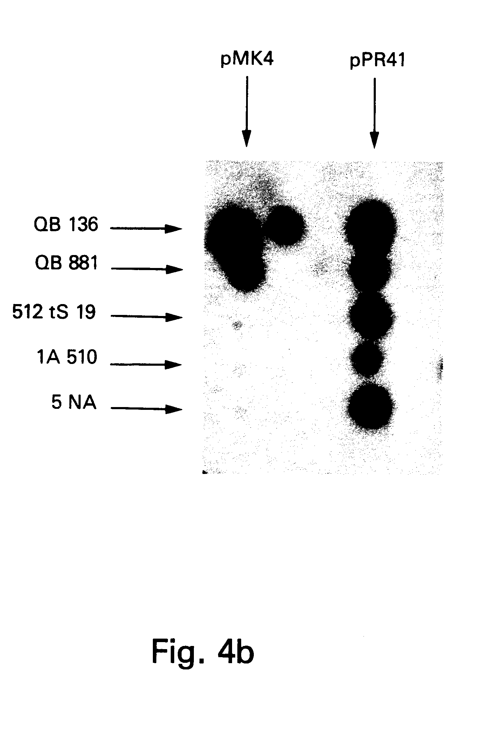

L'activité saccharolytique a été testée en disposant les colonies sur un support à base d'agar mou et contenant du saccharose, et par détection de la présence de glucose après 2 heures d'incubation à 37°C (figure 4b). L'augmentation de l'activite saccharolytique exocellulaire, présentée sur la figure 4b, est due à la levane-saccharase dont la synthèse est induite par le saccharose (Microbiology. Schlessinger, D.(Ed). American Society for Microbiology, Washington D.C, pp 58-69, (1976)).

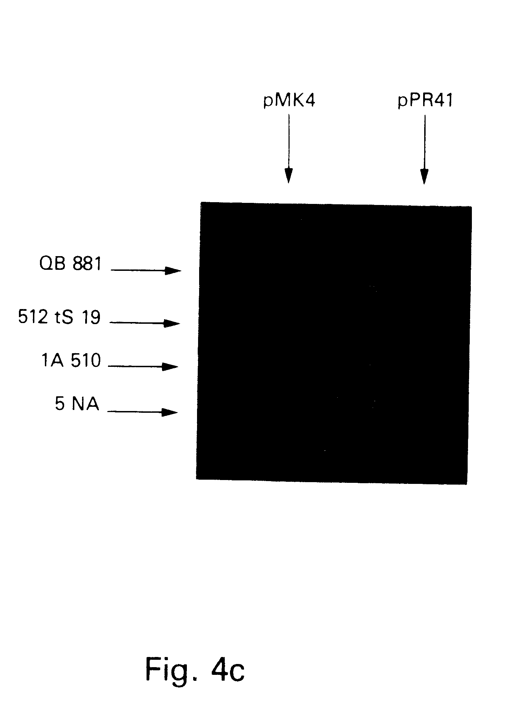

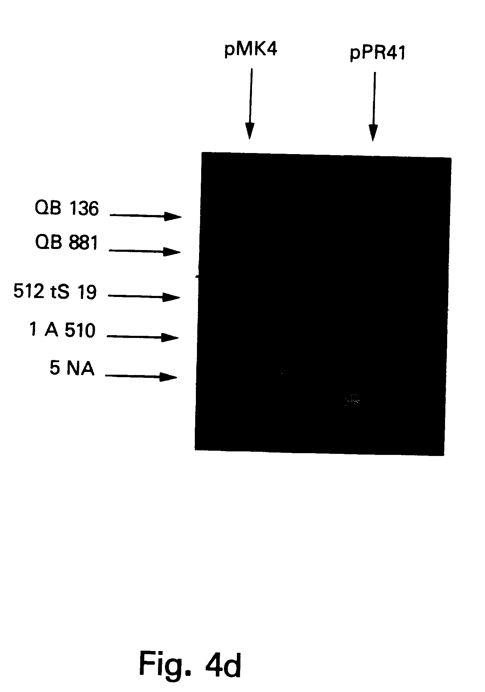

L'activité cellulolytique (figure 4c et 4d) a été testée en cultivant les souches en présence soit de carboxyméthylcellulose (CMC), qui est un glucane $\beta$ 1-4, (figure 4c) soit de lichenane, qui est un mélange de glucanes $\beta$ 1-3 et $\beta$ 1-4 (figure 4d). Puisque le rouge Congo se lie spécifiquement aux glucanes de hauts poids moléculaires, la dégradation de ces deux substrats est indiquée par la présence d'un halo clair autour des colonies. L'hydrolyse de ces deux substrats en présence de pPR41, indique une augmentation de la sécrétion de $\beta$ 1-4 glucanase (J. Bacteriol., 165, 612-619, (1986) et/ou $\beta$-1, 3-1,4-glucanase (Gene, 23, 211-219, (1983). Une augmentation comparable a été obtenue avec les souches porteuses de mutation sacU$^h$.

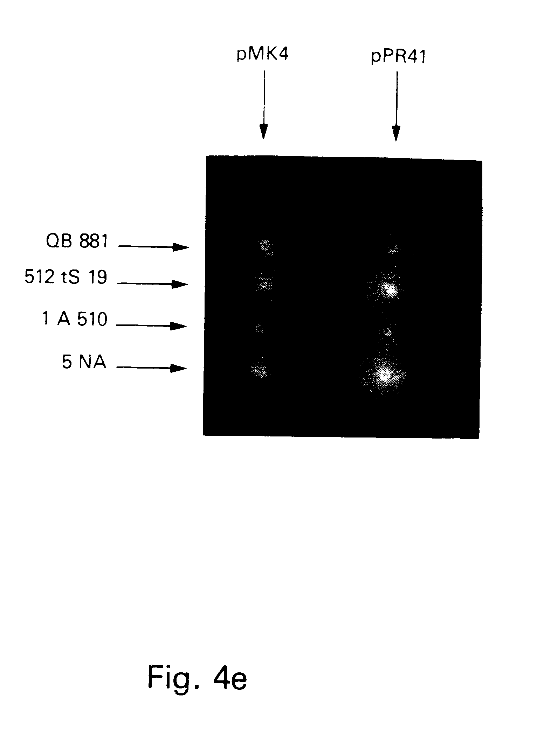

La xylanase est la quatrième enzyme sécrétée et stimulée (figure 4e). Les colonies cultivées en présence de xylane (polymère $\beta$ 1-4) présentent des halos de dégradation plus larges en présence du plasmide pPR41.

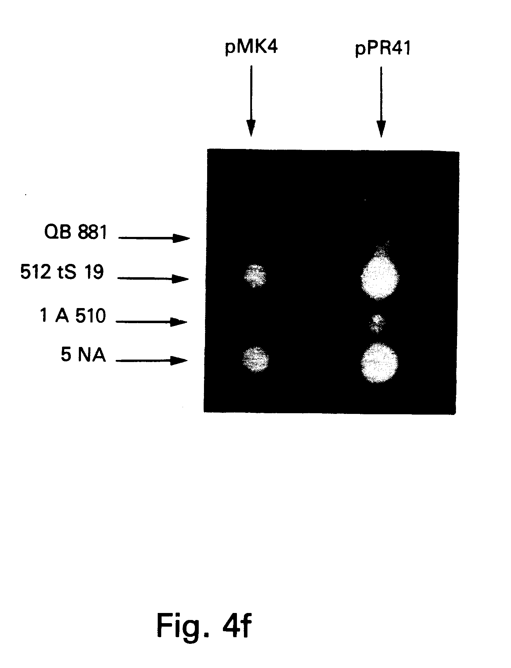

La sécrétion d'un cinquième type d'enzyme de dégradation exocellulaire est augmentée (figure 4f). Les souches cultivées en présence d'amidon en tant qu'unique source de carbone présentent de plus grands halos de dégradation quand pPR41 est présent. L'augmentation, toutefois, n'est pas aussi importante qu'elle ne l'était pour les quatre autres activités exocellulaires.

- sous-clonage du fragment de 1 400 bp

De manière à identifier le plus petit fragment d'ADN capable d'induire une hypersécrétion chez B.subtilis, les fragments obtenus après délétion avec Bal31 utilisés pour le séquençage du phage M13 monobrin ont été transférés au plasmide bifonctionnel pMK4.

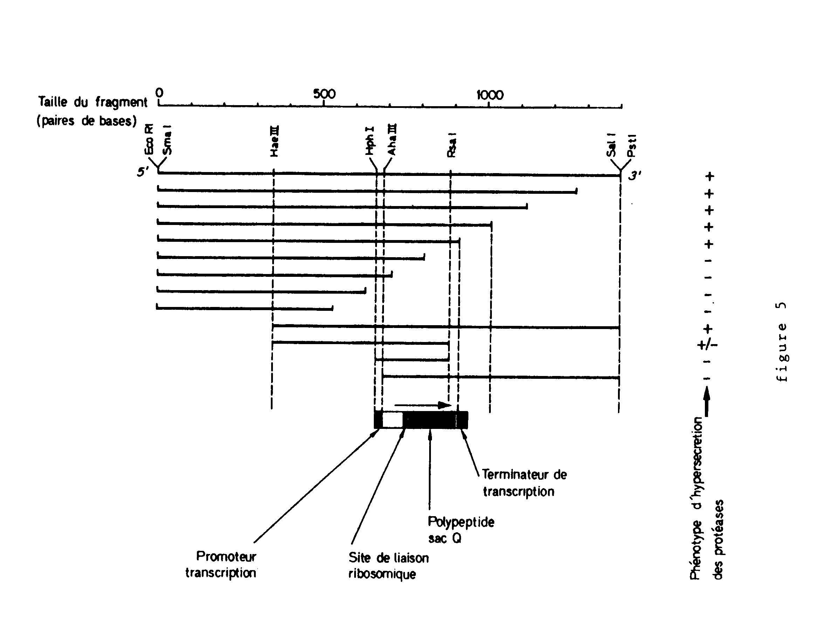

Les plasmides contenant les fragments progressivement délétés à partir de l'extrémité 3'ont été introduits dans la souche de B.subtilis 1A510 et l'activité de type protéase sécrétée a été visualisée sur des milieux solides à base de lait écrémé. Les résultats sont indiqués sur la figure 5.

Le phénotype de l'hypersécrétion est donné par le fragment délété depuis l'extrémité 3'jusqu'en position 914 (fragment ayant perdu les 489 derniers nucléotides) et est perdu lorsque la délétion est étendue jusqu'à 109 paires de base plus loin vers l'extrémité 5', à la position 805.

Il peut ainsi en être conclu que le fragment de 109 paires base compris entre les deux points de délétion porte tout ou partie du fragment d'ADN qui produit le phénotype d'hypersécrétion chez B.subtilis.

Des sites de restriction appropriés ont alors été utilisés pour réduire plus encore la taille du fragment d'ADN à partir de l'extrémité 5'.

Un fragment de 353 paires de bases peut être délété du côté 5'puisque le fragment HaeIII-PstI (position 353-1400) continue d'induire l'hypersécrétion de protéase. Il en a été conclu que la séquence responsable de l'hypersécrétion est comprise entre la position 353 (site HaeIII) et la position 914 (délétion Bal31). Dans ce fragment de 561 paires de base, les 29 derniers nucléotides ne peuvent être délétés sans perte partielle du phénotype. En effet, le sous-clonage du fragment HaeIII-RsaI conduit à une hyperproduction de protéase légèrement diminuée par rapport au fragment initial. Par contre, les 109 derniers nucléotides sont essentiels, puisque leur délétion conduit à la perte totale du phénotype Prt[h].

Ces 109 nucléotides, toutefois, ne semblent pas être suffisants puisque le fragment AhaIII (685)-PstI (1 400) ne contient pas l'information nécessaire pour induire une hypersecrétion.

La conclusion est donc que la taille du fragment produisant le phénotype Ses[h] est inférieure à 561 bp (HaeIII, 353-délétion 914) mais supérieure à 228 bp (AhaIII, 686-délétion 914).

- Identification et caractérisation du polypeptide responsable du phénotype Ses[h] :

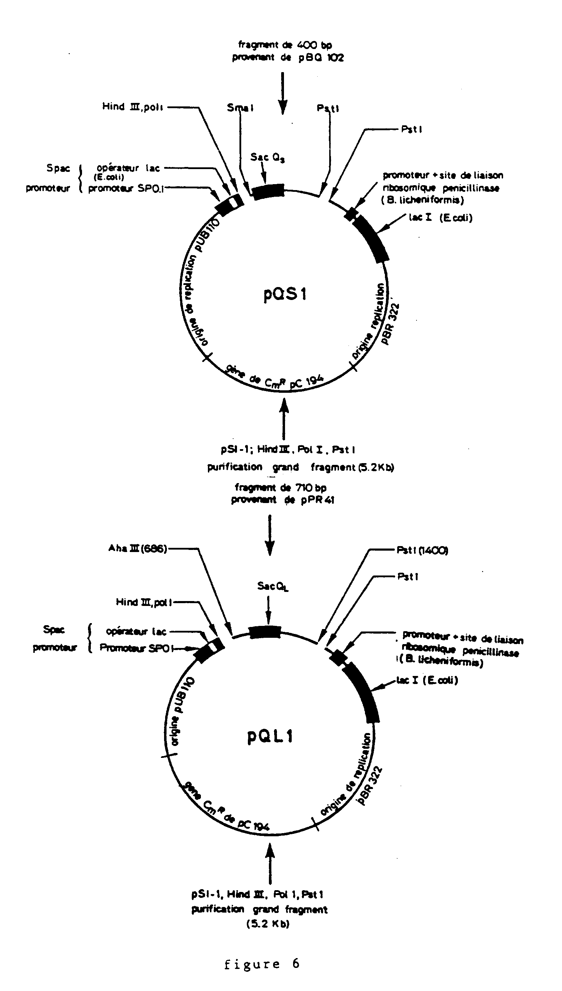

Le fragment AhaIII (686) PstI (1 400) a été cloné sous contrôle d'un promoteur reconnu par B. subtilis en phase végétative de croissance et inductible par l'IPTG (promoteur spac., Proc. Natl. Acad. Sci. U.S.A, 81, 439-443 (1984)). Les plasmides pQL₁ et pQS₁ contenant respectivement les gènes codant pour sacQ$_L$, sous contrôle du même promoteur inductible spac, sont illustrés à la figure 6, est leur construction est plus particulièrement détaillée dans la légende de cette figure à la fin de ce chapitre 2.

Les résultats du tableau 2 montrent que la secrétion de protéase alcaline et de lévane-saccharase est induite spécifiquement par l'IPTG chez B. subtilis 1A510. Ce résultat, combiné aux données de sous-clonage, montre qu'un produit de transcription et/ou de traduction provenant du fragment de 228 bp (AhaIII, 686 délétion 914) suffit à produire le phénotype Ses[h].

*Tableau 2 : Mesure de l'activité excellulaire de la lévane-saccharase et de la protéase alcaline*

| Souche réceptrice | Plasmide | Description de la construction | Lévane-saccharase unités/mg x $10^3$ | | Protéase alcaline unités azocaséine/ml sensibles au PMSF | |
|---|---|---|---|---|---|---|
| | | | - IPTG | + IPTG | - IPTG | + IPTG |
| 1A510 | pMK4 | Contrôle vecteur | < 20 | | 29 | |
| | pBQ1 | pMK4 + sacQ$_S$ | 200 | | 240 | |
| | pPR41 | pMK4 + sacQ$_L$ | 1100 | | 450 | |
| | pSI1 | promoteur spac (contrôle) | < 20 | < 20 | 49 | 49 |
| | pQS1 | promoteur spac + sacQ$_S$ | < 20 | 200 | 40 | 67 |
| | pQL1 | promoteur spac + sacQ$_L$ | < 20 | 1300 | 41 | 290 |
| QB136 (sacU$^h$) | pMK4 | contrôle vecteur | 600 | | 490 | |
| | pBQ1 | pMK4 + sacQ$_S$ | 600 | | 370 | |
| | pPR41 | pMK4 + sacQ$_L$ | 1100 | | 670 | |

Ceci suggère l'intervention d'un polypeptide codé à partir de ce fragment. Il contient en effet une phase ouverte de lecture codant pour un peptide de 46 résidus précédé par une séquence typique de liaison ribomique ($\Delta G$ = -20,4 Kcal/mole, figure 3) et se terminant par une séquence caractéristique de terminaison de transcription ($\Delta G$ = -24,2 Kcal/mole).

Ce peptide présente une homologie significative avec le produit du gène sacQ de B. subtilis (J. Bacteriol., 166, 113-119) et B. amyloliquefaciens (J. Bacteriol., 3, 85-96). Le polypeptide SacQ$_L$ de B.

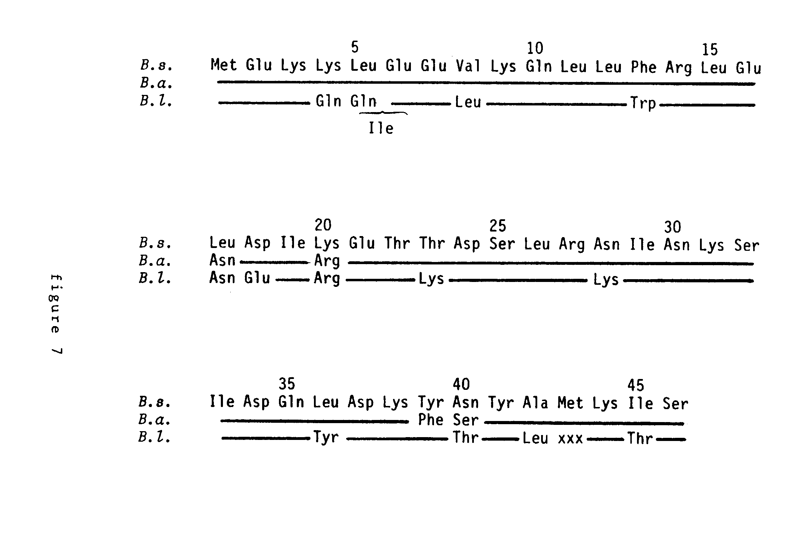

licheniformis a la même taille de 46 acides aminés que son homologue sacQ$_S$ de B. subtilis (figure 7). Néanmoins, 15 résidus sont différents chez B. licheformis dont une insertion et une délétion. Sept changements parmi ces 15, font intervenir une modification de la polarité de l'acide aminé. Le polypeptide sacQ$_L$ se distingue donc, par sa structure primaire, du polypeptide sacQ$_S$, mais aussi du polypeptide sacQ$_A$ (B. amyloliquefaciens), qui ne contient que 4 acides aminés différents par rapport à sacQ$_S$ dont 2 seulement font intervenir un changement de polarité.

Cette distinction se marque aussi dans l'intensité du phénotype d'hypersécrétion. Le tableau 2 montre les activités protéase et lévane-saccharase produites par B. subtilis 1A510 portant le gène sacQ$_L$ ou sacQ$_S$ sur un vecteur présent en multiples copies. Le gène sacQ$_S$ de B. subtilis a été cloné de la même façon que sacQ$_L$. Des fragments supérieurs à 3 kb provenant d'une digestion partielle Sau3A1 du DNA chromosomique de B. subtilis 168 ont été liés au site BamHI de pMK4 et introduits dans B. subtilis 168 après amplification dans E. coli JM83. Un clone hyperproducteur de protéases contenait le plasmide pBQ1. Ce plasmide contient une insertion de 2,9 kb au site BamHI et exprime le phénotype Ses$^h$ semblable à celui produit par pPR41. La taille de l'insertion a été réduite à 0,4 kb par une méthode semblable au sous-clonage de pPR4 : digestion partielle Sau3A1 de pBQ1 et sélection des tailles 0,2-0,5 kb. La séquence de ce fragment de 0,4 kb a été déterminée. Elle est strictement identique à la séquence publiée pour sacQ$_S$.

Le tableau 2 montre que la présence du gène sacQ$_L$ en multiples copies dans la souche 1A510 stimule 5 fois plus l'activité de la lévane-saccharase secrétée que le gène sacQ$_S$ dans les mêmes conditions. Quant à l'activité protéase alcaline (sensible au PMSF), elle est deux fois plus stimulée par le gène de B. licheniformis que par le gène de B. subtilis. Cette stimulation devient jusqu'à trois fois meilleure pour des temps de culture plus longs (40 heures). Le gène sacQ$_L$ produit donc le phénotype d'hypersecrétion (Ses$^h$) chez B. subtilis d'une façon plus efficace que le gène sacQ$_S$. Cette efficacité accrue peut être due à la nature même du polypeptide ou à une augmentation de la quantité de ce polypeptide produit dans B. subtilis.

Les résultats du tableau 2 montrent que la séquence primaire du polypeptide sacQ$_L$ joue un rôle capital dans l'expression du phénotype Ses$^h$. En effet, lorsque les deux gènes sacQ$_L$ et sacQ$_S$ sont placés sous le contrôle du même promoteur spac inductible par l'IPTG (voir constructions figure 6), l'activité lévane-saccharase est 6 fois mieux stimulée par sacQ$_L$ qu'elle ne l'est par sacQ$_S$. L'activité protéase alcaline est environ 10 fois mieux stimulée par l'expression du gène de B. licheniformis.

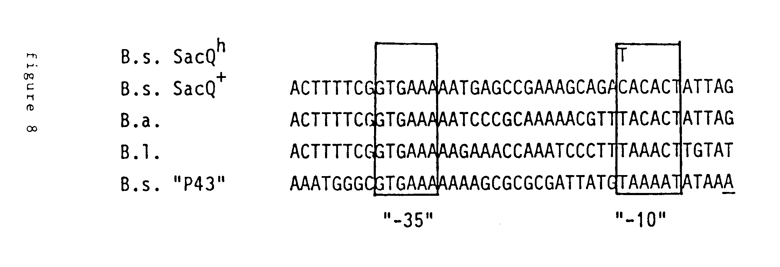

L'activité protéase alcaline est moins stimulée lorsque les gènes sacQ$_L$ ou sacQ$_S$ sont sous le contrôle du promoteur spac que sous contrôle de leur promoteur propre (tableau 2). Il faut donc admettre que la régulation de l'expression du polypeptide sacQ est également importante dans l'expression du phénotype Ses$^h$. Or nous savons d'une part par les résultats de sous-clonage de la figure 5 et ceux du tableau 2 que le promoteur de transcription doit se trouver sur le fragment de 333 paires de bases délimité par les sites HaeIII (353) et AhaIII (386). D'autre part, le site de début de transcription a été déterminé par "S1 mapping" chez B. subtilis (J. Bacteriol., 166, 113-119 (1986)). Par homologie, la position supposée de début de transcription chez B. licheniformis est le nucléotide 695 (figure 3 et figure 8). La région "- 10" par rapport à ce début de transcription ainsi que la région "- 35" présente une séquence qui ressemble à celle d'un promoteur de transcription. Un exemple d'un promoteur (P43) de phase végétative reconnu "in vitro" par la RNA polymérase contenant le facteur $\sigma^{43}$ est donné à titre comparatif (J. Biol. Chem., 259, 8619-8625, (1984)). La figure 8 montre la structure de ce promoteur potentiel comparée dans les différentes souches de Bacillus. La séquence "- 35" est conservée dans toutes les souches alors que la séquence "- 10" est variable. Le premier nucléotide de la séquence "- 10" est important. En effet, la substitution d'un C par un T conduit au phénotype Ses$^h$ dans le mutant SacQ$^h$ de B. subtilis (J. Bacteriol., 166, 113-119, (1986)). Cette même mutation est conservée dans B. amyloliquefaciens et B. licheniformis, qui possèdent tous deux de meilleures capacités de secrétion que B. subtilis. Chez B. licheniformis hyperproducteur de protéases, on trouve une mutation supplémentaire de C en A au troisième nucléotide de la séquence "- 10".

- Effet synergique de sacQ sur la mutation sacU$^h$ :

Le tableau 2 compare les effets de la présence des gènes sacQ$_L$ et sacQ$_S$ en multiples copies dans une souche de B. subtilis QB136 qui porte la mutation sacU$^h$. La présence du gène sacQ$_S$ n'augmente pas le niveau d'activité lévane-saccharase et diminue celui de la protéase alcaline de la souche QB136 alors que la présence du gène sacQ$_L$ augmente environ deux fois ces niveaux d'activité.

Toutes les activités lévane-saccharase présentées dans le tableau 2 ont été effectuées en présence de saccharose. En absence de saccharose, l'activité est inférieure à 0,02 U/mg (résultat non montré).

En d'autres termes, l'induction de l'expression du gène sacB par le saccharose semble être un phénomène de sa surexpression provoquée par la présence de plusieurs copies du gène sacQ.

- La portion carboxyterminale du polypeptide sacQ est hypervariable :

Le fragment HaeIII (353)-RsaI (885) a été sous-cloné au site SmaI du vecteur pMK4 et un phénotype partiel d'hyperproduction de protéase a été obtenu (figure 5). D'après les séquences connues du vecteur pMK4 et du gène sacQ$_L$, on peut prédire la synthèse d'un polypeptide hybride dans lequel les 7 derniers acides aminés de sacQ$_L$ sont remplacés par 11 résidus totalement différents provenant de la traduction d'une séquence de pMK4. Ce polypeptide sacQ hybride contenant la partie carboxy-terminale modifiée exprime donc une activité diminuée par rapport au polypeptide intact. Ceci suggère que les sept derniers résidus contribuent à donner une structure optimale au polypeptide, mais ne sont pas essentiels à son activité.

Ce résultat est compatible avec le fait que les derniers résidus du polypeptide sacQ sont variables dans B. licheniformis (figure 7).

Néanmoins, un polypeptide fusionné qui contient seulement les douze premiers acides aminés du polypeptide SacQ suivis par 19 acides aminés provenant de la lec-ture du vecteur pMK4 est totalement dépourvu d'activité (délétion 805).

- Identification d'une cible du gène sacQ

Afin d'identifier une cible du gène sacQ, les inventeurs ont réalisé une fusion de séquences nucléotidique dans laquelle le site régulateur (sacR) du gène sacB codant pour la lévane-saccharase est utilisé pour contrôler l'expression du gène de structure d'une protéine cytoplasmique, la 3', 5''-aminoglycoside phosphotransférase (aph3') de Streptococcus faecalis (Gene., 23, 331-341 (1983)). Ce gène confère une résistance à la kanamycine lorsqu'il est exprimé dans B. subtilis .

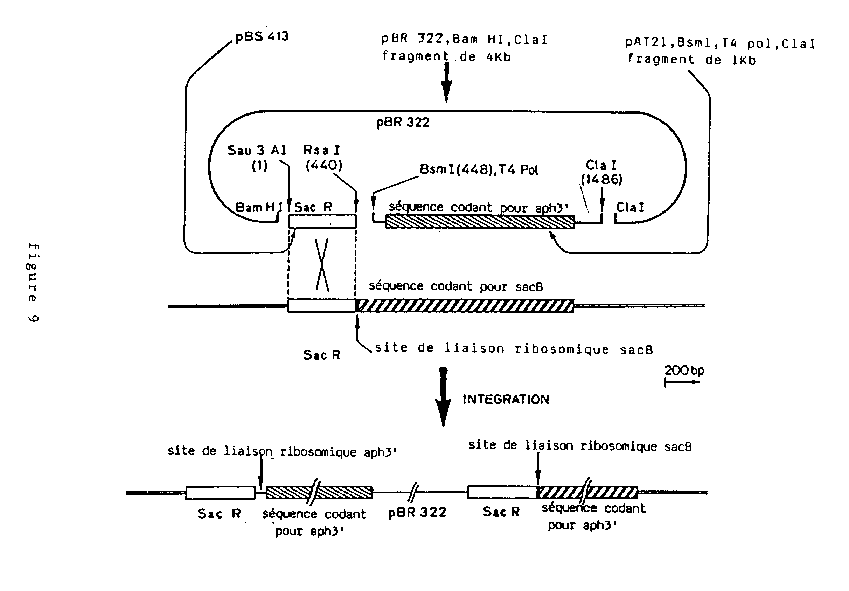

La figure 9 montre la manière dont la souche QB4058 a été construite.

Après intégration par recombinaison homologue, les séquences chromosomiques demeurent théoriquement intactes jusqu'à la fin du locus sacR, au niveau du site Rsa1 440 (nucléotide numéroté comme il l'a été décrit dans Mol. Gen. Genet., 200, 220-228 (1984)) qui est localisé juste avant le site de liaison aux ribosomes du gène sacB.

Ainsi sacR est directement suivi par le site de liaison aux ribosomes du gène aph3', la séquence codant pour l'aph3' et les signaux de terminaison, la séquence pBR322 et finalement les séquences sacR et sacB situées en aval du site Sau3A1 (position 1) de sacR.

Le tableau 3 représente la mesure de la résistance à la kanamycine de la souche de B. subtilis QB4058. La concentration minimale de kanamycine nécessaire pour inhiber la croissance de la souche de B. subtilis QB4058 portant les plasmides indiqués, a été déterminée selon la méthode décrite dans Antibiot. Chemother. 9, 307-311 (1959). Plusieurs dilutions (1 : $10^2$, 1 : $10^3$, ou 1 : $10^4$) d'une culture de QB4058 en croissance exponentielle dans un bouillon PAB (Penassay Broth commercialisé par DIFCO) contenant du chloramphénicol ont été placées sur de l'agar Mueller-Hinton contenant de la kamamycine et, lorsque cela est indiqué, 2 % de saccharose. Le phénotype de secrétion de lévane-saccharase (encore désigné par l'abréviation Lvs) est estimé par détection sur plaque : Lvs$^-$ correspond à l'absence d'halo d'activité saccharolytique ; Lvs$^+$ et Lvs$^-$ correspondent respectivement à la présence d'un petit et d'un grand halo d'activité saccharolytique.

Le tableau 3 montre que la résistance à la kamamycine est conférée à la souche QB4058 par la présence simultanée de multiples copies de sacQ$_S$ et de saccharose. sacQ$_L$ confère une meilleure résistance à l'antibiotique que ne le fait sacQ$_S$ confirmant les résultats préliminaires concernant la meilleure efficacité du polypeptide issu de B. licheniformis.

| PLASMIDE PRESENT DANS LA SOUCHE QB4058 | CONCENTRATION MINIMALE EN KANAMYCINE ($\mu$g/ml) REQUISE POUR L'INHBITION DE CROISSANCE | | PHENOTYPE DE DE SECRETIONS DE LEVANE-SACCHARASE | |
|---|---|---|---|---|
| | -SACCHAROSE | + SACCHAROSE | -SACCHAROSE | + SACCHAROSE |
| pMK4 | 8 | 16 | Lvs⁻ | Lvs⁺ |
| pBQ1 (pMK4 + sacQs) | 8 | 32 | Lvs⁻ | Lvsʰ |
| pPR41 (pMK4 + sacQl) | 8 | 128 | Lvs⁻ | Lvsʰ |

Ainsi, l'expression du gène aph 3' est activée par surproduction du polypeptide sacQ.

Il peut être également conclu de cette expérience que le site d'activation est un fragment d'ADN localisé en amont du site RsaI (440) qui comprend sacR. De manière à déterminer à quelle distance la cible de sacQ est localisée en amont du site RsaI, l'activité de secrétion de lévane-saccharase par les souches résistantes à la kamamycine a été testée.

Le tableau 3 montre que le niveau de secrétion de lévane-saccharase est augmenté en présence de plasmides porteurs du gène sacQ.

La cible de sacQ doit donc se située en aval du site Sau3A1. Par conséquent, la cible sacQ semble se trouver dans le fragment Sau3AI-RsaI localisé au voisinnage de la séquence codant pour la lévane-saccharase.

- Exemples de construction d'un plasmide contenant la séquence signal de la lévane-saccharase

a) Construction du plasmide pBS610

L'ADN extrait à partir d'une souche de B.subtilis168 a été partiellement digéré avec HindIII.

Après électrophorèse sur gel de polyacrylamide, les fragments de 4 kb ont été clonés dans le plasmide pUC8, décrit dans Gene, 19, 259-268 (1982), qui ne se réplique que chez E.coli. Le plasmide recombinant a été isolé à partir du clone synthétisant de la lévane-saccharase. Ce dernier a été détecté par son action sur le saccharose, qui est transformé en glucose et en polymères de fructose sous l'action de la lévane-saccharase.

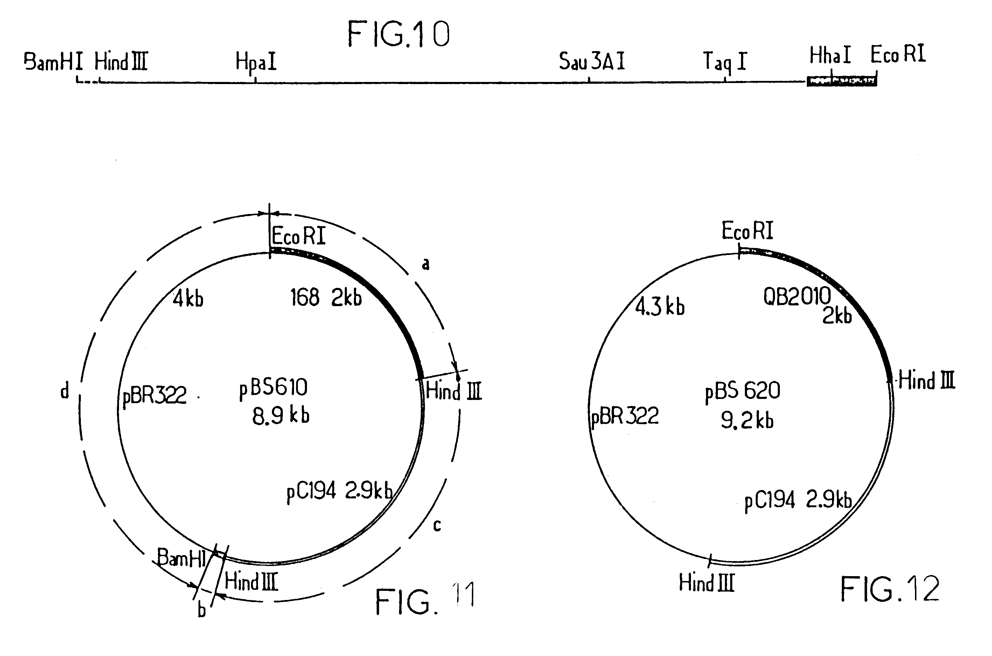

Le plasmide isolé a été digéré avec EcoRI et BamHI. Le fragment BamHI-EcoRI qui a été isolé, comprend un fragment HindIII-EcoRI d'environ 2 kb et dont la carte de restriction est indiquée à la figure 10. Ce fragment contient la séquence signal de la lévane-saccharase.

Le fragment BamHI-EcoRI sus-mentionné a été recombiné, à l'intérieur d'un nouveau plasmide, avec le fragment BamHI-EcoRI obtenu à partir de pBR322, après digestion de ce dernier avec les enzymes BamHI et EcoRI, et contenant les éléments responsables pour la réplication du pBR322 chez E.coli.

Un fragment HindIII-HindIII d'environ 2,9 kb, consistant en le plasmide pC194, et comprenant les éléments permettant audit plasmide pC194 de se répliquer chez B.subtilis, a été inséré dans le plasmide recombinant précédemment obtenu, plus particulièrement au niveau du site HindIII contenu dans le fragment BamHI-EcoRI comprenant la séquence signal de la lévane-saccharase, pour finalement obtenir le plasmide pBS610 schématiquement représenté à la figure 11. Le plasmide pBS610, dont le génome est d'environ 8,9 kb, peut se répliquer chez E.coli et chez B. subtilis.

Le plasmide pC194 peut être obtenu à partir du plasmide pHV33. Une souche d'E.coli transformée par pHV33 a été déposée à la Collection Nationale des Cultures de Micro-organismes (C.N.C.M) à l'Institut Pasteur, France, sous le numéro I-191.

b) Construction du plasmide pBS620

Un fragment HindIII-EcoRI, contenant la séquence signal de la lévane-saccharase, a été obtenu par digestion en présence des endonucléases EcoRI et HindIII du phage $\lambda$ modifié obtenu par insertion, au niveau des sites BamHI du phage $\lambda$-EMBL3 décrit dans J. Mol. Biol., 170, 827-842 (1983), de produits de digestion partielle Sau3A1 de l'ADN de la souche de B. subtilis2010 porteuse d'une mutation sacRᶜ et secrétant de la lévane-saccharase de façon permanente. Le phage modifié sus-mentionné comprend le promoteur sacRᶜ susdit. Une souche d'E.coli contenant ce phage modifié a été déposée à la C.N.C.M sous

le numéro I-314.

Le fragment EcoRI-HindIII de 2 kb contenant la séquence signal de la lévane-saccharase et le locus sacR$^c$ ont été recombinés avec le fragment EcoRI-HindIII d'environ 4,3 kb isolé à partir de pBR322 et contenant les éléments permettant au plasmide pBR322 de se répliquer chez E.coli. Le plasmide obtenu a encore été digéré avec HindIII et recombiné avec le fragment de 2,9 kb sus-mentionné, provenant du plasmide pC194, afin d'obtenir le plasmide pBS620 schématisé à la figure 12. Le pBS620 peut également se répliquer chez B. subtilis et E.coli.

Les plasmides pBS610 et pBS620 peuvent ensuite être modifiés par insertion d'une séquence d'ADN codant pour un polypeptide déterminé, de préférence à la suite de la séquence signal, et par insertion en amont de ladite séquence signal, d'une séquence d'ADN comprenant une séquence cible selon l'invention et les éléments permettant l'expression de la séquence signal et de la séquence d'ADN codant pour le polypeptide déterminé par la cellule hôte.

D'une manière générale la modification des plasmides comprenant une séquence signal de manière à ce qu'ils comportent une séquence d'ADN codant pour un polypeptide déterminé, peut être réalisée par toute méthode susceptible d'être imaginée par l'homme de l'art en fonction notamment des divers enzymes et sites de restriction choisis.

Les plasmides modifiés ainsi obtenus peuvent être utilisés pour transformer B. subtilis de manière à ce que ce dernier produise et excrète le polypeptide déterminé. Cette transformation peut par exemple être réalisée selon la méthode décrite dans J. Bacteriol., 114, 273-286 (1973). Les cellules contenant des plasmides et résistant à l'antibiotique peuvent être sélectionnées sur des plaques d'agar SP additionnées de chloramphénicol (5 µg/ml).

Les cellules de B. subtilis transformées peuvent être détectées par exemple en mettant en jeu des anticorps préalablement préparés et dirigés contre le polypeptide déterminé. Les cellules des colonies détectées peuvent alors être isolées, et utilisées pour préparer des cultures de cellules synthétisant et sécrétant le polypeptide déterminé dans le milieu de culture. Le polypeptide peut ensuite être isolé du milieu de culture par toute méthode appropriée.

Les légendes correspondant aux figures illustrant les expériences et observations qui précèdent sont les suivantes :

- figure 1 :

Bacillus subtilis 1A510 contenant le vecteur pHV33 ou le plasmide pPR4 sont mis en culture à 37°C en milieu SP supplémenté par du chloramphénicol à 5 µg/ml et de la caséine à 1 %. L'activité protéase des surnageants est mesurée en présence ou en absence de PMSF aux temps indiqués.

- figure 2 :

Plasmide pPR41. Amp$^R$ et Cm$^R$ indiquent les gènes de résistance à l'ampicilline et au chloramphénicol, respectivement. Ce plasmide contient une origine de réplication de E. coli et une origine de réplication de B. subtilis.

- figure 3 :

Séquence du fragment de 1 400 paires de bases. Les points de délétion Bal31 sont indiqués par ⌐. Les sites reconnus par les enzymes de restriction sont soulignés ainsi qu'une séquence palindromique suggérant la structure d'un terminateur de transciption. Le début de transcription probable est indiqué par →. La séquence complémentaire à l'extrémité 3' de l'ARN ribosomique 16s de B. subtilis est souligné d'un double trait.

- figure 4a :

Test qualitatif pour l'activité protéolytique secrétée par B. subtilis. Les souches sont cultivées pendant 3 jours sur des boîtes de Pétri contenant du milieu MM supplémenté par du chloramphénicol à 5 µg/l et de la poudre de lait écrémé (Difco) à 2 %.

- figure 4b :

Tests qualitatifs de l'activité saccharolytique dans B. subtilis. Après une croissance de nuit en milieu SP contenant du chloramphénicol, les boîtes sont pulvérisées avec 1 ml de saccharose à 50 %. Après 1 heure 30 d'incubation à 37°C, le glucose libéré est visualisé par révélation avec du Statzyme.

- figure 4c :

Tests qualitatifs de dégradation de la carboxyméthylcellulose, les colonies sont mises en culture de nuit en milieu L contenant du chloramphénicol. Après recouvrement par de l'agar mou contenant 1 % de carboxyméthylcellulase, elles sont incubées pendant 4 heures à 37°C avant révélation avec du Rouge Congo.

- figure 4d :

Tests qualitatifs de dégradation de lichenane. Les colonies sont mises en culture pendant 2 jours en milieu MM supplémenté par du $(NH_4)_2SO_4$ à 2 %, de l'hydrolysat de caséine 0,01 %, du chloramphénicol à 5 $\mu$g/ml et du lichenane à 0,4 %, avant révélation au Rouge Congo.

- figure 4e :

Test qualitatif de dégradation de xylane. Les colonies sont mises en culture à 37°C pendant 2 jours sur milieu MM supplémenté par du $(NH_4)_2 SO_4$ à 2 %, de l'hydrolysat de caséine à 0,01 %, du chloramphénicol à 5 $\mu$g/ml et du xylane à 0,4 % avant révélation au Rouge Congo.

- figure 4f :

Tests qualitatifs de dégradation de l'amidon. Les colonies sont mises en culture pendant 3 jours sur milieu MM supplémenté par du $(NH_4)_2 SO_4$ à 2 %, de l'hydrolysat de caséine à 0,01 %, du chloramphénicol à 5 $\mu$g/ml et de l'amidon à 1 %. Les boîtes sont révélées à l'iode.

- figure 5 :

Sous-clonage du fragment de 1 400 paires de bases.

- figure 6 :

Construction de plasmides contenant sacQ$_L$ et sacQ$_S$ sous contrôle du même promoteur inductible spac.

Les plasmides pQL1 et pQS1 sont construits par insertion de fragments portant les gènes sacQ$_L$ et sacQ$_S$ dépourvus de leur promoteur dans le plasmide pSI-1 au site HindIII après remplissage à la DNA polymérase (polI). Le plasmide pSI-1 contient le promoteur spac introduit au site EcoRI de pIQ45 (Proc. Natl. Acad. U.S.A, 81, 439-443 (1984)). La position du site AhaIII et PstI indiquée pour pQL1 correspond aux positions de la séquence de la figure 3. Le plasmide pBQ102 provient du sous-clonage d'un fragment de 400 paires de bases au site BamHI de pMK4. Ce fragment a été obtenu par digestion partielle par Sau3A1 du plasmide pBQ1. La séquence de ce fragment montre qu'il commence au site Sau3A1 correspondant à la position 47 de la séquence sacQ de B. subtilis publiée (J. Bacteriol., 166, 113-119 (1986)). Le début de la séquence du fragment inséré dans pQS1 est donc G-G-G-G-A-T-C-T-T-T-C-A-A-A...

Le site Sau3A1 (G-A-T-C) souligné correspond au site Sau3A1 indiqué à la figure 3.

- figure 7 :

Comparaison des polypeptides sacQ de B. subtilis (B. s.), B. amyloliquefaciens (B. a.) et B. licheniformis (B.a.) et B. licheniformis (B.l.). Une ligne continue indique des résidus identiques à ceux de B. subtilis. Les acides aminés différents sont indiqués pour B. amyloliquefaciens et B. liqueniformis. Une délétion est indiquée au XXX et une insertion par . Les séquences pour B. s. et B. a. sont celles publiées (J. Bacteriol., 166, 113-119, (1986)).

- figure 8 :

Comparaison des structures de promoteurs possibles dans les différentes souches de Bacillus. Les séquences suivantes sont publiées : B. subtilis SacQ$^h$, sacQ$^+$ : J. Bacteriol., 166, 113-119, (1986) ; B. amyloliquefaciens : J. Biotechnol., 3, 85-96, (1985) ; B. subtilisP43 : J. Biol. Chem., 259, 8619-8625, (1984).

Les abréviations B. s, B. a et B. l correspondent respectivement à B. subtilis, B. amyloliquefaciens et B. licheniformis.

- figure 9 :

Construction du gène de fusion sacR-aph3'. Le plasmide utilisé pour l'intégration par recombinaison homologue a été construit par ligature in vitro des 3 fragments sacR, aph3' et sacB. La numérotation nucléotidique est identique à celle publiée dans Mol. Gen. Genet., 200, 220-228 (1985) pour sacR, et dans Gene., 23, 331-341 (1983) pour aph3'. Le plasmide pBS413 a été cloné comme suit. Un produit de digestion partielle par HindIII de l'ADN chromosomique de B. subtilis 168 a été ligaturé au niveau du site HindIII de pUC 8. Une souche d' E. coli transformée par pBS413, dans lequel a été inséré un fragment de 4,1 kb provenant d'une digestion partielle par HindIII contenant sacB, présente une activité saccharolytique. Un fragment EcoRI de 2,0 kb contenant sacR et le début de sacB a été purifié à partir de pBS413 et utilisé pour isoler le fragment Sau3A1-Rsa1 de 440 paires de bases contenant sacR. Le plasmide pAT21 a été décrit dans Gene., 23, 331-341 (1983). Le dérivé pBR322 contenant le gène de fusion a été introduit dans le chromosone de B. subtilis168 par intégration homologue. Une souche contenant le gène de fusion (QB4058) a été sélectionnée sur des plaques de kanamycine contenant 2 % de saccharose. Le site d'intégration, déterminé par des expériences de transduction pBS1, correspond au locus sacB.

- Figure 10 :

Carte de restriction du fragment HindIII-EcoRI issu de la souche de B.subtilis168. Ce fragment contient, au niveau du trait plus épais de la figure, la séquence signal de la lévane-saccharase ainsi que les 112 premiers nucléotides du gêne codant pour la lévane-saccharase.

- Figure 11 :

Plasmide pBS610.

Les parties indiquées par les arcs de cercle a et b (d'environ 2 kb et 20 paires de base respectivement) proviennent du fragment BamHI-EcoRI comprenant le fragment HindIII-EcoRI de la figure 10, la partie indiquée par l'arc de cercle c (d'environ 2,9 kb) provient du plasmide pC194 et la partie d (d'environ 4 kb) provient de pBR322.

- Figure 12 :

Plasmide pBS620.

Les différentes parties de pBS620 sont indiquées sur la figure avec les références des plasmides ou des ADN à partir desquels elles ont été respectivement obtenues.

La souche de B. subtilis QB4058 a été déposée à la C.N.C.M à l'Institut Pasteur à Paris sous le numéro I 587.

La souche de B.subtilis transformée par le plasmide pPR41 a été déposée à la C.N.C.M à l'Institut Pasteur de Paris sous le numéro I-579.

Toute séquence présentant la capacité de s'hybrider avec les séquences actives décrites ci-dessus, et capable d'induire le même type d'activité, entre dans le cadre de la protection conférée à l'invention par les revendications qui suivent.

Ainsi, toute séquence qui ne se distinguerait de celles qui ont été plus particulièrement décrites, que par un certain nombre de mutations ne modifiant pas les propriétés stimulantes du mutant, sont à considérer comme des équivalents des séquences plus spécifiquement identifiées dans les revendications.

**Revendications**

**Revendications pour les Etats contractants suivants : BE, CH, DE, ES, FR, GB, GR, IT, LI, LU, NL, SE**

1. Fragment d'ADN issu du gène sacQ de B.licheniformis caractérisé en ce qu'il code pour le polypeptide ayant la structure suivante :

```
(Nterminal)Met-Glu-Lys-Gln-Gln-Ile-Glu-Glu-Leu-Lys-Gln-
Leu-Leu-Trp-Arg-Leu-Glu-Asn-Glu-Ile-Arg-Glu-Thr-Lys-
Asp-Ser-Leu-Arg-Lys-Ile-Asn-Lys-Ser-Ile-Asp-Gln-Tyr-
Asp-Lys-Tyr-Thr-Tyr-Leu-Lys-Thr-Ser(Cterminal),
```

ou pour tout fragment de ce polypeptide qui conserve la propriété du polypeptide susdit d'activer l'expression du phénotype d'hypersecrétion Ses$^h$ conférée par ledit polypeptide chez B.subtilis.

2. Fragment d'ADN selon la revendication 1 caractérisé en ce qu'il consiste en un polynucléotide délimité par les nucléotides d'extrémité correspondant aux nucléotides situés aux positions 768 et 905 respectivement de la figure 3.

3. Fragment d'ADN issu d'un fragment Sau3A1 de 3,5 kb de B.licheniformis caractérisé en ce qu'il ne contient pas de séquence nucléotidique codant pour tout ou partie d'une protéase alcaline, et en ce qu'il comprend un fragment d'ADN selon la revendication 1 ou 2, précédé par une séquence nucléotidique comprenant les éléments de régulation de l'expression de ce dernier.

4. Fragment d'ADN selon la revendication 3, caractérisé en ce que la séquence nucléotidique comprenant les éléments de régulation de l'expression du fragment d'ADN, selon la revendication 1 ou 2, est celle associée à ce dernier dans le génome naturel de B.licheniformis.

5. Fragment d'ADN selon la revendication 3 ou la revendication 4, caractérisé en ce qu'il consiste en tout polynucléotide délimité d'une part par un nucléotide compris entre les positions 1 à 353 et, d'autre part, par un nucléotide compris entre les positions 905 à 914 de la figure 3.

6. Fragment d'ADN selon la revendication 3 ou 4, caractérisé en ce que le fragment d'ADN selon la revendication 1 ou 2 est suivie par une séquence nucléotidique comprenant les éléments terminateurs de la transcription de ce dernier.

20

7. Fragment d'ADN selon la revendication 3, caractérisé en ce que la séquence nucléotidique comprenant les éléments terminateurs de la transcription du fragment d'ADN selon la revendication 1 ou 2, est celle associée à ce dernier dans le génome naturel de B.licheniformis.

8. Fragment d'ADN selon la revendication 6 ou 7, caractérisé en ce qu'il consiste en tout polynucléotide délimité d'une part par un nucléotide compris entre les positions 1 à 353 et, d'autre part, par un nucléotide compris entre les positions 1012 à 1400 de la figure 3.

9. Acide nucléique recombinant contenant le fragment d'ADN selon l'une quelconque des revendications 1 à 8 inséré dans un acide nucléique hétérologue vis-à-vis dudit fragment d'ADN.

10. Acide nucléique recombinant selon la revendication 9 caractérisé en ce qu'il consiste en un plasmide contenant ledit fragment d'ADN en l'un de ses sites non essentiels pour sa réplication.

11. Plasmide selon la revendication 10 caractérisé en ce qu'il s'agit d'un plasmide utilisable pour la transformation de B.subtilis.

12. Plasmide selon la revendication 11 caractérisé en ce qu'il consiste en un plasmide navette entre B.subtilis et E.coli.

13. Plasmide selon l'une quelconque des revendication 10 à 12, caractérisé en ce qu'il possède une origine de réplication lui conférant la propriété de se diviser en multicopies dans la cellule hôte.

14. Plasmide selon l'une quelconque des revendication 10 à 13, caractérisé en ce qu'il contient un promoteur inductible.

15. Cellules de B.subtilis, productrices de quantités accrues d'enzymes exocellulaires caractérisées en ce qu'elles contiennent un plasmide selon l'une quelconque des revendications 10 à 14, capable de se répliquer dans lesdites cellules.

16. Cellules de B.subtilis selon la revendication 15, caractérisées en ce qu'elles comportent également une mutation sacU$^h$.

17. Procédé de production d'enzymes exocellulaires, caractérisé par la mise en culture de cellules d'une lignée de B.subtilis selon la revendication 15 ou 16 et par la récupération desdites enzymes, à partir du milieu de culture.

18. Polypeptide activateur de l'expression du phénotype d'hypersecrétion conférée par ledit polypeptide de Ses$^h$, caractérisé par la structure suivante:

```
(Nterminal)Met-Glu-Lys-Gln-Gln-Ile-Glu-Glu-Leu-Lys-Gln-
Leu-Leu-Trp-Arg-Leu-Glu-Asn-Glu-Ile-Arg-Glu-Thr-Lys-
Asp-Ser-Leu-Arg-Lys-Ile-Asn-Lys-Ser-Ile-Asp-Gln-Tyr-
Asp-Lys-Tyr-Thr-Tyr-Leu-Lys-Thr-Ser(Cterminal).
```

**Revendications pour l'Etat contractant suivant : AT**

1. Procédé de production d'enzymes exocellulaires caractérisé par la mise en culture de cellules d'une lignée de B. subtilis contenant un plasmide contenant un fragment d'ADN issu du gène B. licheniformis codant pour le polypeptide ayant la structure suivante :

```
(Nterminal)Met-Glu-Lys-Gln-Gln-Ile-Glu-Glu-Leu-Lys-Gln-
Leu-Leu-Trp-Arg-Leu-Glu-Asn-Glu-Ile-Arg-Glu-Thr-Lys-Asp-
Ser-Leu-Arg-Lys-Ile-Asn-Lys-Ser-Ile-Asp-Gln-Tyr-Asp-Lys-
Tyr-Thr-Tyr-Leu-Lys-Thr-Ser(Cterminal)
```

ou pour tout fragment de ce polypeptide qui conserve la propriété du polypeptide susdit d'activer l'expression du phénotype d'hypersécrétion Ses[h] conférée par ledit polypeptide chez B. subtilis et par la récupération desdites enzymes à partir du milieu de culture.

2. Procédé de production d'enzymes exocellulaires selon la revendication 1 caractérisé en ce que le fragment d'ADN consiste en un polynucléotide délimité par les nucléotides d'extrémité correspondant aux nucléotides situés aux positions 768 et 905 respectivement de la figure 3.

3. Procédé de production d'enzymes exocellulaires selon la revendication 1 ou 2 caractérisé en ce que le fragment d'ADN issu d'un fragment Sau3A1 de 3,5 kb de B. licheniformis et en ce qu'il ne contient pas de séquence nucléotidique codant pour tout ou partie d'une protéase alcaline, et en ce qu'il comprend un fragment d'ADN selon la revendication 1 ou 2, précédé par une séquence nucléotidique comprenant les éléments de régulation de l'expression de ce dernier.

4. Procédé de production d'enzymes exocellulaires selon la revendication 3 caractérisé en ce que la séquence nucléotidique comprenant les éléments de régulation de l'expression du fragment d'ADN, selon la revendication 1 ou 2, est celle associée à ce dernier dans le génome naturel de B. licheniformis.

5. Procédé de production d'enzymes exocellulaires selon revendication 3 ou 4 caractérisé en ce qu'il consiste en tout polynucléotide délimité d'une part par un nucléotide compris entre les positions 1 à 353 et, d'autre part, par un nucléotide compris entre les positions 905 et 914 de la figure 3.

6. Procédé de production d'enzymes exocellulaires selon la revendication 3 ou 4 caratérisé en ce que le fragment d'ADN selon la revendication 1 ou 2 est suivie par une séquence nucléotidique comprenant les éléments terminateurs de la transcription de ce dernier.

7. Procédé de production d'enzymes exocellulaires caractérisé en ce que la séquence nucleotidique comprenant les éléments terminateurs de la transcription du fragment d'ADN selon la revendication 1 ou 2, est celle associée à ce dernier dans le génome naturel de B. licheniformis.

8. Procédé de production d'enzymes exocellulaires selon la revendication 6 ou 7 caractérisé en ce qu'il consiste en tout polynucléotide délimité d'une part par un nucléotide compris entre les positions 1 à 353 et, d'autre part, par un nucléotide compris entre les positions 1012 à 1400 de la figure 3.

9. Procédé de production d'enzymes exocellulaires caractérisé en ce que l'acide nucléique recombinant contenant le fragment d'ADN selon l'une quelconque des revendications 1 à 8 est inséré dans un acide nucléique hétérologue vis-à-vis dudit fragment d'ADN.

10. Procédé de production d'enzymes exocellulaires selon la revendication 1 caractérisé en ce que le plasmide contient ledit fragment d'ADN en l'un de ses sites non essentiels pour sa réplication.

11. Procédé de production d'enzymes exocellulaires selon la revendication 10 caractérisé en ce qu'il s'agit d'un plasmide utilisable pour la transformation de B. subtilis.

12. Procédé de production d'enzymes exocellulaires selon la revendication 11 caractérisé en ce qu'il consiste en un plasmide navette entre B. subtilis et E. coli.

13. Procédé de production d'enzymes exocellulaires selon l'une quelconque des revendications 10 à 12, caractérisé en ce qu'il possède une origine de réplication lui conférant la propriété de se diviser en multicopies dans la cellule hôte.

**14.** Procédé de production d'enzymes exocellulaires selon l'une quelconque des revendications 10 à 13, caractérisé en ce qu'il contient un promoteur inductible.

**15.** Procédé de production d'enzymes exocellulaires selon la revendication 1, caractérisé en ce que le cellule comporte également une mutation sacU$^h$.

**16.** Procédé de production d'un polypeptide tel que défini dans la revendication 1 qui comprend la production d'un polypeptide déterminé par une souche de B. subtilis dont le génome a été modifié par insertion d'une séquence nucléotidique codant pour ledit polypeptide qui comprend la transformation de la souche B. subtilis sus-indiquée, au moyen de vecteurs permettant la surproduction d'un activateur du phénotype Ses$^h$ dans les cellules de B. subtilis, la mise en culture desdites cellules transformées dans un milieu approprié, et la récupération dudit polypeptide à partir du milieu de culture.

**Claims**

**Claims for the following Contracting States : BE, CH, DE, ES, FR, GB, GR, IT, LI, LU, NL, SE**

**1.** DNA fragment obtained from the B. licheniformis sacQ gene, characterized in that it encodes the polypeptide having the following structure:

```
(Nterminal)Met-Glu-Lys-Gln-Gln-Ile-Glu-Glu-Leu-Lys-Gln-
Leu-Leu-Trp-Arg-Leu-Glu-Asn-Glu-Ile-Arg-Glu-Thr-Lys-
Asp-Ser-Leu-Arg-Lys-Ile-Asn-Lys-Ser-Ile-Asp-Gln-Tyr-
Asp-Lys-Tyr-Thr-Tyr-Leu-Lys-Thr-Ser(Cterminal),
```

or any fragment of this polypeptide which preserves the property of the abovementioned polypeptide of activating the expression of the hypersecretion phenotype Ses$^h$ conferred by the said polypeptide in B.subtilis.

**2.** DNA fragment according to Claim 1 characterized in that it consists of a polynucleotide delimited by the end nucleotides corresponding to the nucleotides situated at positions 768 and 905 respectively of Figure 3.

**3.** DNA fragment obtained from a 3.5 kb Sau3A1 fragment from B. licheniformis, characterized in that it does not contain a nucleotide sequence encoding all or part of an alkaline protease, and in that it comprises a DNA fragment according to Claim 1 or 2, preceded by a nucleotide sequence comprising the regulatory elements for the expression of the said fragment.

**4.** DNA fragment according to Claim 3, characterized in that the nucleotide sequence comprising the regulatory elements for the expression of the DNA fragment, according to Claim 1 or 2, is that associated with the latter in the natural B. licheniformis genome.

**5.** DNA fragment according to Claim 3 or Claim 4, characterized in that it consists of any polynucleotide delimited, on the one hand, by a nucleotide between positions 1 to 353 and, on the other hand, by a nucleotide between positions 905 and 914 of Figure 3.

**6.** DNA fragment according to Claim 3 or 4, characterized in that the DNA fragment according to Claim 1 or 2 is followed by a nucleotide sequence comprising the elements for termination of the transcription of the said fragment.

**7.** DNA fragment according to Claim 3, characterized in that the nucleotide sequence comprising the elements for termination of the transcription of the DNA fragment according to Claim 1 or 2, is that associated with the latter in the natural B. licheniformis genome.

**8.** DNA fragment according to Claim 6 or 7, characterized in that it consists of any polynucleotide delimited, on the one hand, by a nucleotide between positions 1 to 353 and, on the other hand, by a nucleotide between positions 1012 to 1400 of Figure 3.

9. Recombinant nucleic acid containing the DNA fragment according to any one of Claims 1 to 8, inserted into a nucleic acid which is heterologous with respect to the said DNA fragment.

10. Recombinant nucleic acid according to Claim 9, characterized in that it consists of a plasmid containing the said DNA fragment in one of its sites which are non-essential for its replication.

11. Plasmid according to Claim 10, characterized in that it is a plasmid which can be used for the transformation of B. subtilis.

12. Plasmid according to Claim 11, characterized in that it consists of a shuttle plasmid between B. subtilis and E. coli.

13. Plasmid according to any one of Claims 10 to 12, characterized in that it has a replication origin which confers on it the property of dividing into multiple copies in the host cell.

14. Plasmid according to any one of Claims 10 to 13, characterized in that it contains an inducible promoter.

15. B. subtilis cells, which are producers of enhanced quantities of exocellular enzymes, characterized in that they contain a plasmid according to any one of Claims 10 to 14, which is capable of replicating in the said cells.

16. B. subtilis cells according to Claim 15, characterized in that they also contain a $sacU^h$ mutation.

17. Process for the production of exocellular enzymes, characterized by the culturing of cells of a B. subtilis line according to Claim 15 or 16 and by recovering the said enzymes, from the culture medium.

18. Polypeptide for activation of the expression of the hypersecretion phenotype $Ses^h$ conferred by the said polypeptide, characterized by the following structure:

```
(Nterminal)Met-Glu-Lys-Gln-Gln-Ile-Glu-Glu-Leu-Lys-Gln-
Leu-Leu-Trp-Arg-Leu-Glu-Asn-Glu-Ile-Arg-Glu-Thr-Lys-
Asp-Ser-Leu-Arg-Lys-Ile-Asn-Lys-Ser-Ile-Asp-Gln-Tyr-
Asp-Lys-Tyr-Thr-Tyr-Leu-Lys-Thr-Ser(Cterminal).
```

**Claims for the following Contracting State : AT**

1. Process for the production of exocellular enzymes, characterized by the culturing of cells of a B. subtilis line containing a plasmid containing a DNA fragment obtained from the B. licheniformis gene encoding the polypeptide having the following structure:

```
(Nterminal)Met-Glu-Lys-Gln-Gln-Ile-Glu-Glu-Leu-Lys-Gln-
Leu-Leu-Trp-Arg-Leu-Glu-Asn-Glu-Ile-Arg-Glu-Thr-Lys-Asp-
Ser-Leu-Arg-Lys-Ile-Asn-Lys-Ser-Ile-Asp-Gln-Tyr-Asp-Lys-
Tyr-Thr-Tyr-Leu-Lys-Thr-Ser(Cterminal),
```

or any fragment of this polypeptide which preserves the property of the abovementioned polypeptide of activating the expression of the hypersecretion phenotype $Ses^h$ conferred by the said polypeptide in B.subtilis and by recovering the said enzymes from the culture medium.

2. Process for the production of exocellular enzymes according to Claim 1, characterized in that the DNA fragment consists of a polynucleotide delimited by the end nucleotides corresponding to the nucleotides situated at positions 768 and 905 respectively of Figure 3.

3. Process for the production of exocellular enzymes according to Claim 1 or 2, characterized in that the DNA fragment is obtained from a 3.5 kb Sau3A1 fragment from B. licheniformis and in that it does not contain a nucleotide sequence encoding all or part of an alkaline protease, and in that it comprises a DNA fragment according to Claim 1 or 2, preceded by a nucleotide sequence comprising the regulatory elements for the expression of the said fragment.

4. Process for the production of exocellular enzymes according to Claim 3, characterized in that the nucleotide sequence comprising the regulatory elements for the expression of the DNA fragment, according to Claim 1 or 2, is that associated with the latter in the natural B. licheniformis genome.

5. Process for the production of exocellular enzymes according to Claim 3 or 4, characterized in that it consists of any polynucleotide delimited, on the one hand, by a nucleotide between positions 1 to 353 and, on the other hand, by a nucleotide between positions 905 and 914 of Figure 3.

6. Process for the production of exocellular enzymes according to Claim 3 or 4, characterized in that the DNA fragment according to Claim 1 or 2 is followed by a nucleotide sequence comprising the elements for termination of the transcription of the said fragment.

7. Process for the production of exocellular enzymes, characterized in that the nucleotide sequence comprising the elements for termination of the transcription of the DNA fragment according to Claim 1 or 2, is that associated with the latter in the natural B. licheniformis genome.

8. Process for the production of exocellular enzymes according to Claim 6 or 7, characterized in that it consists of any polynucleotide delimited, on the one hand, by a nucleotide between positions 1 to 353 and, on the other hand, by a nucleotide between positions 1012 to 1400 of Figure 3.

9. Process for the production of exocellular enzymes, characterized in that the recombinant nucleic acid containing the DNA fragment according to any one of Claims 1 to 8 is inserted into a nucleic acid which is heterologous with respect to the said DNA fragment.

10. Process for the production of exocellular enzymes according to Claim 1, characterized in that the plasmid contains the said DNA fragment in one of its sites which are non-essential for its replication.

11. Process for the production of exocellular enzymes according to Claim 10, characterized in that it is a plasmid which can be used for the transformation of B. subtilis.

12. Process for the production of exocellular enzymes according to Claim 11, characterized in that it consists of a shuttle plasmid between B. subtilis and E. coli.

13. Process for the production of exocellular enzymes according to any one of Claims 10 to 12, characterized in that it has a replication origin which confers on it the property of dividing into multiple copies in the host cell.

14. Process for the production of exocellular enzymes according to any one of Claims 10 to 13, characterized in that it contains an inducible promoter.

15. Process for the production of exocellular enzymes according to Claim 1, characterized in that the cell also contains a sacU$^h$ mutation.

16. Process for the production of a polypeptide as defined in Claim 1, which comprises the production of a determined polypeptide by a B. subtilis strain whose genome has been modified by insertion of a nucleotide sequence encoding the said polypeptide which comprises the transformation of the abovementioned B. subtilis strain, by means of vectors permitting the overproduction of an activator of the Ses$^h$ phenotype in the B. subtilis cells, the culturing of the said transformed cells in an appropriate

25

medium, and the recovering of the said polypeptide from the culture medium.

**Patentansprüche**
**Patentansprüche für folgende Vertragsstaaten : BE, CH, DE, ES, FR, GB, GR, IT, LI, LU, NL, SE**

1. DNA-Fragment aus dem Gen sacQ von B. licheniformis, **dadurch gekennzeichnet**, daß es für das Polypeptid der folgenden Struktur codiert:

(N-terminal)Met-Glu-Lys-Gln-Gln-Ile-Glu-Glu-Leu-Lys-Gln-Leu-Leu-Trp-Arg-
Leu-Glu-Asn-Glu-Ile-Arg-Glu-Thr-Lys-Asp-Ser-Leu-Arg-Lys-Ile-Asn-Lys-Ser-
Ile-Asp-Gln-Tyr-Asp-Lys-Tyr-Thr-Tyr-Leu-Lys-Thr-Ser(C-terminal),

oder für irgendein Fragment dieses Polypeptids, welches die Eigenschaft des genannten Polypeptids, die Expression des Hypersekretions-Phenotyps Ses[h], die durch das Polypeptid an B. subtilis verliehen wird, zu aktivieren, beibehält.

2. DNA-Fragment nach Anspruch 1, **dadurch gekennzeichnet**, daß es aus einem Polynucleotid besteht, welches durch die End-Nucleotide begrenzt wird, die den Nucleotiden entsprechen, die in den Positionen 768 bzw. 905 der Figur 3 angeordnet sind.

3. DNA-Fragment aus einem Fragment Sau3A1 mit 3,5 kb von B. licheniformis, **dadurch gekennzeichnet**, daß es keine Nucleotidsequenz enthält, die für eine vollständige oder einen Teil einer alkalischen Protease codiert, und daß es ein DNA-Fragment nach Anspruch 1 oder 2 umfaßt, welchem eine Nucleotidsequenz vorausgeht, die Elemente zur Steuerung der Expression dieses letzteren umfaßt.

4. DNA-Fragment nach Anspruch 3, **dadurch gekennzeichnet**, daß die Nucleotidsequenz, welche die Elemente der Steuerung der Expression des DNA-Fragments gemäß Anspruch 1 oder 2 umfaßt, jene ist, die in dem natürlichen Genom von B. licheniformis mit dem letzteren assoziiert ist.

5. DNA-Fragment nach Anspruch 3 oder Anspruch 4, **dadurch gekennzeichnet**, daß es aus jeglichem Polynucleotid besteht, welches einerseits durch ein zwischen den Positionen 1 bis 353 liegendes Nucleotid und andererseits durch ein zwischen den Positionen 905 und 914 der Figur 3 liegendes Nucleotid begrenzt wird.

6. DNA-Fragment nach Anspruch 3 oder 4, **dadurch gekennzeichnet**, daß das DNA-Fragment nach Anspruch 1 oder 2 von einer Nucleotidsequenz gefolgt wird, die Terminationselemente der Transkription dieses letzteren umfaßt.

7. DNA-Fragment nach Anspruch 3, **dadurch gekennzeichnet**, daß die Terminationselemente der Transkription des DNA-Fragments nach Anspruch 1 oder 2 enthaltende Nucleotidsequenz jene ist, die in dem natürlichen Genom von B. licheniformis mit diesem letzteren assoziiert ist.

8. DNA-Fragment nach Anspruch 6 oder 7, **dadurch gekennzeichnet**, daß es aus jeglichem Polynucleotid besteht, welches einerseits durch ein zwischen den Positionen 1 bis 353 liegendes Nucleotid und andererseits durch ein zwischen den Positionen 1012 bis 1400 der Figur 3 liegendes nucleotid begrenzt wird.

9. Rekombinante Nucleinsäure enthaltend das DNA-Fragment nach einem der Ansprüche 1 bis 8, eingefügt in eine gegenüber dem DNA-Fragment heterologe Nucleinsäure.

10. Rekombinante Nucleinsäure nach Anspruch 9, **dadurch gekennzeichnet**, daß sie aus einem Plasmid besteht, welches das DNA-Fragment in einer seiner Stellungen enthält, die für seine Replikation nicht essentiell ist.

11. Plasmid nach Anspruch 10, **dadurch gekennzeichnet**, daß es sich um ein für die Transformation von B. subtilis geeignetes Plasmid handelt.

12. Plasmid nach Anspruch 11, **dadurch gekennzeichnet**, daß es aus einem Transport-Plasmid zwischen B. subtilis und E. coli besteht.

13. Plasmid nach einem der Ansprüche 10 bis 12, **dadurch gekennzeichnet**, daß es einen Replikations-Ursprung besitzt, der ihm die Eigenschaft verleiht, sich in der Wirtszelle in Vielfachkopien aufzuteilen.

14. Plasmid nach einem der Ansprüche 10 bis 13, **dadurch gekennzeichnet**, daß es einen induzierbaren Promotor enthält.

15. Zellen von B. subtilis, die erhöhte Mengeen von exozellulären Enzymen bilden, **dadurch gekennzeichnet**, daß sie ein Plasmid nach einem der Ansprüche 10 bis 14 enthalten, welches dazu in der Lage ist, sich in den genannten Zellen zu vermehren.

16. Zellen von B. subtilis nach Anspruch 15, **dadurch gekennzeichnet**, daß sie weiterhin eine Mutation sacU$^h$ umfassen.

17. Verfahren zur Herstellung von exozellulären Enzymen, **dadurch gekennzeichnet**, daß man Zellen einer Zellinie von B. subtilis nach Anspruch 15 oder 16 züchtet und die genannten Enzyme aus dem Kulturmedium gewinnt.

18. Polypeptid, welches die Expression des Hypersekretions-Phenotyps aktiviert, der durch das Polypeptid von Ses$^h$ verliehen wird, **gekennzeichnet durch** die folgende Struktur:

(N-terminal)Met-Glu-Lys-Gln-Gln-Ile-Glu-Glu-Leu-Lys-Gln-Leu-Leu-Trp-Arg-

Leu-Glu-Asn-Glu-Ile-Arg-Glu-Thr-Lys-Asp-Ser-Leu-Arg-Lys-Ile-Asn-Lys-Ser-

Ile-Asp-Gln-Tyr-Asp-Lys-Tyr-Thr-Tyr-Leu-Lys-Thr-Ser(C-terminal).

**Patentansprüche für folgenden Vertragsstaat : AT**

1. Verfahren zur Herstellung von exozellulären Enzymen, **dadurch gekennzeichnet**, daß man Zellen einer Zellinie von B. subtilis, die ein Plasmid enthalten, welches ein DNA-Fragment aus dem Gen von B. licheniformis enthält, welches für das Polypeptid der folgenden Struktur codiert:

(N-terminal)Met-Glu-Lys-Gln-Gln-Ile-Glu-Glu-Leu-Lys-Gln-Leu-Leu-Trp-Arg-

Leu-Glu-Asn-Glu-Ile-Arg-Glu-Thr-Lys-Asp-Ser-Leu-Arg-Lys-Ile-Asn-Lys-Ser-

Ile-Asp-Gln-Tyr-Asp-Lys-Tyr-Thr-Tyr-Leu-Lys-Thr-Ser(C-terminal)

oder für irgendein Fragment dieses Polypeptids, welches die Eigenschaft des genannten Peptids, die Expression des Hypersekretions-Phenotyps Ses$^h$, die durch das Polypeptid an B. subtilis verliehen wird, zu aktivieren, beibehält, züchtet, und die genannten Enzyme aus dem Kulturmedium gewinnt.

2. Verfahren zur Herstellung von exozellulären Enzymen nach Anspruch 1, **dadurch gekennzeichnet**, daß das DNA-Fragment aus einem Polynucleotid besteht, welches durch die Endnucleotide begrenzt wird, die den Nucleotiden entsprechen, die in den Positionen 768 bzw. 905 der Figur 3 angeordnet sind.

3. Verfahren zur Herstellung von exozellulären Enzymen nach Anspruch 1 oder 2, **dadurch gekennzeichnet**, daß das DNA-Fragment aus einem Fragment Sau3A1 mit 3,5 kb von B. licheniformis hervorgegangen ist und keine Nucleotidsequenz enthält, die für eine ganze oder einen Teil einer alkalischen Protease codiert, und daß es ein DNA-Fragment nach Anspruch 1 oder 2 umfaßt, welchem eine Nucleotidsequenzvorausgeht, die Elemente zur Steuerung der Expression dieses letzteren umfaßt.

**4.** Verfahren zur Herstellung von exozellulären Enzymen nach Anspruch 3, **dadurch gekennzeichnet**, daß die Nucleotidsequenz, welche die Elemente der Steuerung der Expression des DNA-Fragments nach Anspruch 1 oder 2 umfaßt, jene ist, die mit diesem letzteren in dem natürlichen Genom von B.licheniformis assoziiert ist.

**5.** Verfahren zur Herstellung von exozellulären Enzymen nach Anspruch 3 oder 4, **dadurch gekennzeichnet**, daß es aus jeglichem Polynucleotid besteht, das einerseits durch ein zwischen den Positionen 1 bis 353 liegendes Nucleotid und andererseits durch ein zwischen den Positionen 905 und 914 der Figur 3 liegendes Nucleotid begrenzt wird.

**6.** Verfahren zur Herstellung von exozellulären Enzymen nach Anspruch 3 oder 4, **dadurch gekennzeichnet**, daß das DNA-Fragment nach Anspruch 1 oder 2 von einer Nucleotidsequenz gefolgt wird, die Terminationselemente der Transkription dieses letzteren umfaßt.

**7.** Verfahren zur Herstellung von exozellulären Enzymen, **dadurch gekennzeichnet**, daß die Terminationselemente der Transkription des DNA-Fragments nach Anspruch 1 oder 2 enthaltende Nucleotidsequenz in dem natürlichen Genom von B. licheniformis mit diesem letzteren assoziiert ist.

**8.** Verfahren zur Herstellung von exozellulären Enzymen nach Anspruch 6 oder 7, **dadurch gekennzeichnet**, daß es aus jeglichem Polynucleotid besteht, welches einerseits durch ein zwischen den Positionen 1 bis 353 liegendes Nucleotid und andererseits durch ein zwischen den Positionen 1012 bis 1400 der Figur 3 liegendes Nucleotid begrenzt wird.

**9.** Verfahren zur Herstellung von exozellulären Enzymen, **dadurch gekennzeichnet**, daß die rekombinante Nucleinsäure, die das DNA-Fragment nach einem der Ansprüche 1 bis 8 enthält, in eine bezüglich des DNA-Fragments heterologe Nucleinsäure eingefügt wird.

**10.** Verfahren zur Herstellung von exozellulären Enzymen nach Anspruch 1, **dadurch gekennzeichnet**, daß das Plasmid das DNA-Fragment in einer seiner für seine Replikation nicht essentiellen Stelle enthält.

**11.** Verfahren zur Herstellung von exozellulären Enzymen nach Anspruch 10, **dadurch gekennzeichnet**, daß es sich um ein für die Transformation von B. subtilis geeignetes Plasmid handelt.

**12.** Verfahren zur Herstellung von exozellulären Enzymen nach Anspruch 11, **dadurch gekennzeichnet**, daß es aus einem Transport-Plasmid zwischen B. subtilis und E. coli besteht.

**13.** Verfahren zur Herstellung von exozellulären Enzymen nach einem der Ansprüche 10 bis 12, **dadurch gekennzeichnet**, daß es einen Replikations-Ursprung umfaßt, der ihm die Eigenschaft verleiht, sich in der Wirtszelle in Vielfachkopien aufzuteilen.

**14.** Verfahren zur Herstellung von exozellulären Enzymen nach einem der Ansprüche 10 bis 13, **dadurch gekennzeichnet**, daß es einen induzierbaren Promotor enthält.

**15.** Verfahren zur Herstellung von exozellulären Enzymen nach Anspruch 1, **dadurch gekennzeichnet**, daß die Zelle weiterhin eine Mutation sacU$^h$ umfaßt.

**16.** Verfahren zur Herstellung eines Polypeptids, wie es in Anspruch 1 definiert ist, umfassend die Bildung eines Polypeptids, welches durch einen Stamm von B. subtilis bestimmt wird, dessen Genom durch Einfügen einer Nucleotidsequenz modifiziert ist, welche für das Polypeptid codiert und welches die Transformation des genannten Stammes von B. subtilis mit Vektoren, die die Überproduktion eines Aktivators des Phenotyps Ses$^h$ in Zellen von B. subtilis umfaßt, das Züchten dieser transformierten Zellen in einem geeigneten Medium und das Gewinnen des Polypeptids aus dem Kulturmedium.

figure 1

figure 2

EcoRI
Sma

GATCGAATGAGAGAACGATCAAGCTGCCGGACGCCGTTTGCCTGCAGGACCGTGGGCGG

GAAAGCCTGCTTAAAATTCTCTGTCGTCCGGATCCAGCGATCAAAGCTCTCCGGCTTTTCTTTGGTTGCCTTTGGGCACGCTATATTTCCTGCCCAAAGTACCGCCAGGCT

TGGATGGCATCCAAAAAGCCAATGAGGAGGAAGCCAATATGAACACCTCTTTCAATTTTACGGGTCAAAAATTGGCCGGATAGACCTTCGGGCCGTTTGAACTTCGGGCCGAT

GGCGTCTCGGGTCTATTGCCGTGTTTTCGGGCGGGCCATACCCGATCCCGTTCGGGTGTTTTGCGGCTCAAGGGCATGGGTTTTGATAAAGGGCCGAAAT

ACACAACTGAACTTGGCATGATAAAAGGGGTTTATATGGATCTTTATTGGACTCTCCAGTTACTTTAGAGACTCAAGGTTGCAAAACCAACAAA
500

TGGGTGTTATGGTCTATTTAAAGTTGCGGGTGTAACCAAGTTTCGGTGAAAAAGAACCAAATCCTTTAAACTTGTATTACAGATCAAATACCTATGACTCG
400

TTCACTATACACAAATTGATTCTTCCAAAAAGGAGTGTGGCAACCGGTGGAAAAGCAACAAAATTGAAGAATTAAAACAACTGCTTTGGCGCTAGAGAATGAAAATCAGAGAAACAAAGG

SerLeuArgLysIleAsnLysSerIleAspGlnTyrAspLysTyrTyrThrTyrLeuLysThrSer
ACTCCTTGCGCAAGAATTAACAATGACTACACTACAATATCTAAAACCTCGTAAAAAGACTTGTTCAGACAAGTCTTTTTTTGTGCAAACGAACCCAAAAAA

fMet GluLysGlnIleGluGluLeuLysGlnLeuLeuTrpArgLeuGluAsnGluIleArgGluThrLysAsp
AGGATAAATTATTATATAAGTTGTAAATAAAAAAATTGCTCTCTGTCAATCGGGAATTTGATTTAGTAAATATTAAGAAAGGAGGACCACCATCTCTAAGCTTTTTGTCTAGATCACAG
1000

TAAAATCAACGAAAGAATGAATCATTTTATAGACCTTGAGAATTCCAATGGGACTTAAATCGGACGCCTGCACGGTTTCTAGATGCAAAGGACCAGTGCATTCAGCGAGCTTGCTTT
1100

TTATCATTACCAAAAGTTTCGGGCGGGCCACACGGACACCGATGCGGCACAAAACCCTGGACGAAGCAATTGAACTCTTGATTCTGCGTTGATTTAGAGGATGAAGACACAGCC
1300

CGATGAACCTTGCGATGAAAATCAACCGTTCAGTCGCAATGAATGCGGCGACTGCGCCTTTATACAATAAGCATTAAAGT

figure 3

31

pMK4    pPR41

QB 136 →

QB 881 →

512 tS 19 →

1A 510 →

5 NA →

# Fig. 4a

pMK4         pPR41

QB 136 ——————→

QB 881 ——————→

512 tS 19 ——————→

1A 510 ——————→

5 NA ——————→

# Fig. 4b

pMK4          pPR41

QB 881 ——→

512 tS 19 ——→

1A 510 ——→

5 NA ——→

Fig. 4c

Fig. 4d

Fig. 4e

pMK4          pPR41

QB 881

512 tS 19

1 A 510

5 NA

Fig. 4f

figure 5

figure 6

figure 7

```
                              5                    10                   15
B.s.    Met Glu Lys Lys Leu Glu Glu Val Lys Gln Leu Leu Phe Arg Leu Glu
B.a.    ─────────────────────────────────────────────────────────────────
B.l.    ──────────── Gln Gln ──────── Leu ─────────────── Trp ───────────
                          Ile

                              20                   25                   30
B.s.    Leu Asp Ile Lys Glu Thr Thr Asp Ser Leu Arg Asn Ile Asn Lys Ser
B.a.    Asn ──────── Arg ─────────────────────────────────────────────────
B.l.    Asn Glu ──── Arg ──────── Lys ─────────────── Lys ────────────────

                              35                   40                   45
B.s.    Ile Asp Gln Leu Asp Lys Tyr Asn Tyr Ala Met Lys Ile Ser
B.a.    ──────────────────────── Phe Ser ─────────────────────────────────
B.l.    ──────────────── Tyr ──────────── Thr ──── Leu xxx ──── Thr ──────
```

figure 8

B.s. SacQ[h]

B.s. SacQ[+]   ACTTTTCGGTGAAAAATGAGCCGAAAGCAGACACACTATTAG

B.a.        ACTTTTCGGTGAAAAATCCCGCAAAAACGTTTACACTATTAG

B.1.        ACTTTTCGGTGAAAAAGAAACCAAATCCCTTTAAACTTGTAT

B.s. "P43"  AAATGGGCGTGAAAAAAAGCGCGCGATTATGTAAAATATAAA

"-35"         "-10"

41

figure 9

# FIG.10

EP 0 261 009 B1

BamHI  HindⅢ        HpaI                          Sau3AI        TaqI      HhaI  EcoRI

FIG. 11

FIG.12